# EUROPEAN PATENT APPLICATION

(11) **EP 4 434 542 A1**
(43) Date of publication of application: **25.09.2024**
(21) Application number: 23382270.9
(22) Date of filing: 23.03.2023
(51) Int. Cl.: A61K 39/12, A61P 31/14, C07K 14/005

(54) **SARS-COV-2 IMMUNOGENIC POLYPEPTIDES AND USES THEREOF**

(71) Applicant: Fundació Privada Institut de Reserca de la Sida-Caixa (IrsiCaixa), 08916 Badalona, Barcelona (ES); Barcelona Supercomputing Center - Centro Nacional de Supercomputación (BSC-CMS), 08034 Barcelona (ES); Institut de Recerca i Tecnologia Agroalimentaires (IRTA), 08140 Caldes de Montbui (Barcelona) (ES)
(72) Inventor: Clotet Sala, Bonaventura, E-08916 Badalona, Barcelona (ES); Blanco Arbues, Julian M., E-08916 Badalona, Barcelona (ES); Carrillo Molina, Jorge, E-08916 Badalona, Barcelona (ES); Ávila Nieto, Carlos, E-08916 Badalona, Barcelona (ES); Amengual Rigo, Pep, E-63067 Offenbach am Main, Hessen (DE); Guallar, Victor, E-17832 Esponella (ES); Segalés Coma, Joaquim, E-08193 Bellaterra, Barcelona (ES); Vergara Alert, Júlia, E-08193 Bellaterra, Barcelona (ES)
(74) Representative: ABG Intellectual Property Law, S.L.

(57) **Abstract**

The present invention relates to new polypeptides, more specifically to immunogenic polypeptides derived from protein Spike of SARS-CoV-2. It also relates to the use of the said polypeptides in eliciting an immune response, preventing or treating a disease caused by the infection by SARS-CoV-2.

## Description

### FIELD OF THE INVENTION

The present invention is comprised within the field of biomedicine. It specifically relates to polypeptides and polynucleotides, and the use thereof in the prevention or treatment of a disease caused by SARS-CoV-2 infection.

### BACKGROUND OF THE INVENTION

Coronaviruses (CoV) are enveloped viruses with a positive-stranded RNA genome. An outbreak of severe acute respiratory syndrome (SARS) occurred in 2002 infecting over 8000 people with about 10 percent fatality rate. A novel coronavirus was identified as the agent causing SARS and named SARS-CoV. Another coronavirus, the Middle East respiratory syndrome coronavirus (MERS-CoV) was identified in 2012, which infected over 2500 people in 27 countries with about 35 percent fatality rate. In December 2019, a novel beta-coronavirus related to MERS-CoV and SARS-CoV initially designated 2019-nCoV and later named SARS-CoV-2 was identified in Wuhan, China. The WHO has declared SARS-CoV-2 a public health emergency of international concern (PHEIC), and since March 2020, characterized the situation as a global pandemic. The disease caused by SARS-CoV-2 is designated COVID-19 and severely affects the health of millions of patients globally, causing an ever-increasing number of deaths around the world.

Although the native protein structure of SARS-CoV-2 is known, it displays a poor immunogenicity. For these reasons, there is an urgent need of immunogenic compounds and vaccines against SARS-CoV-2. Most vaccines are based on a stabilized version of the Spike glycoprotein that include the K986P and V987P mutations. These mutations affect the structural flexibility in the monomers of the native SARS-CoV-2 S trimer that affects its stability and immunogenicity. In addition to pre- and post-fusion spike protein conformations, each Receptor binding domain (RBD) displays a dynamic equilibrium between open (up) and closed (down) configurations. In this regard, there is a need for spike protein variants with a preference for adopting the closed state, and thus, showing limited opening motion and RBD exposure to develop a more stable versions of the spike glycoprotein as well as more efficient methods for their recombinant production.

### SUMMARY OF THE INVENTION

The object of the present invention is to provide variants of the SARS-CoV-2 S glycoprotein (spike protein) which are stabilized in the closed state, thereby improving its immunogenic properties and thereby resulting in increased protection efficacy against the development of SARS-CoV-2 infection. In addition, another object of the present invention is to provide variants of the SARS-CoV-2 Spike glycoprotein that, when expressed in a heterologous expression system, can be produced with higher yield.

The authors of the present invention have developed, by way of extensive experimentation, a set of variants of the SARS-CoV-2 spike protein that surprisingly show improved stability. In addition, the authors of the present invention have also developed a set of variants of the SARS-CoV-2 spike that, when expressed in a recombinant system, result in unexpectedly high yields of the recombinant S trimer.

Thus, in a first aspect, the present invention relates to a polypeptide comprising a region from a SARS-CoV-2 spike protein containing at least one mutation selected from the group consisting of: V987H, L849K, S758E, K947R, Q755R, T961E, V963E, D985L, S982F, L517Y, G416R, K386R, V987W, D427I, D420R, A372W and D420Y, relative to the SARS-CoV-2 spike protein sequence as defined in SEQ ID NO: 1, or a functionally equivalent or immunogenically active variant thereof.

In further aspects, the present invention relates to a polynucleotide encoding the polypeptide of the invention, to a vector comprising the polynucleotide of the invention and to a host cell comprising the vector of the invention.

In another aspect, the invention relates to a trimeric polypeptide formed by the polypeptides of the invention.

In another aspect, the invention relates to a method of producing a polypeptide according to the first aspect of the invention, comprising: expressing in a suitable expression system a polynucleotide according to the second aspect of the invention and recovering the polypeptides from the expression system.

In another aspect, the invention relates to a polynucleotide of the invention, a trimeric polypeptide of the invention or a vector of the invention for use in the prevention or treatment of a disease caused by the infection by SARS-CoV-2.

In another aspect, the invention relates to an immunogenic composition or a vaccine comprising the polypeptide according to the first aspect of the invention, the polynucleotide according to the third aspect of the invention, or the vector according to the third aspect of the invention and a pharmaceutically suitable carrier or excipient.

In another aspect, the invention relates to a method for the detection of the presence of antibodies specific for the SARS-CoV-2 spike protein in a sample which comprises contacting the sample with polypeptide of the invention and, detecting the formation of a complex between antibodies specific for the SARS-CoV-2 spike protein present in the sample and the polypeptide or trimeric polypeptide of the first step of this method.

In another aspect, the invention relates to a method for detecting whether a subject has suffered an infection by SARS-CoV-2 comprising the detection of antibodies against the SARS-CoV-2 spike protein in a sample of the subject by a method as defined in the eightth aspect of the invention.

In another aspect, the invention relates to a method for producing a population of antibodies against SARS-CoV-2, said method comprising administering the polypeptide according to the first aspect of the invention, into a subject so that an immune response against said polypeptides or oligomers is elicited in said subject and; isolating from said subject antibodies, antibody fragments and/or antibody-producing cells recognizing the polypeptides of step (i).

### BRIEF DESCRIPTION OF THE FIGURES

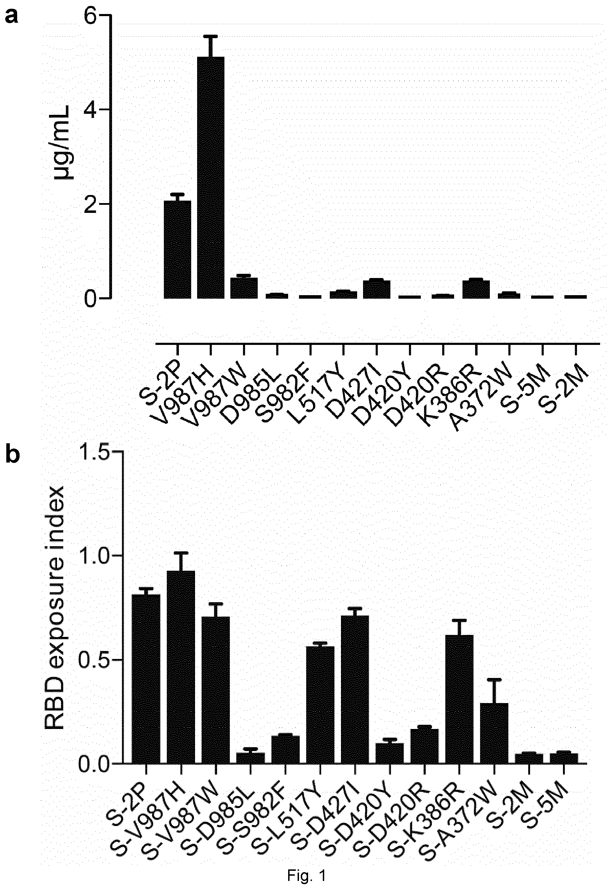

**Figure 1**: Selection of mutations that stabilized the Spike glycoprotein in a closed conformation.. **a)** Levels of recombinant proteins in a 5-day cell culture supernatant of transiently transfected Expi293F cells. Mean plus standard deviation of three experiments are shown. **b)** RBD exposure index in selected recombinant proteins. Data are shown as ratio between RBD binding and total protein. Mean plus standard deviation of three experiments are shown.

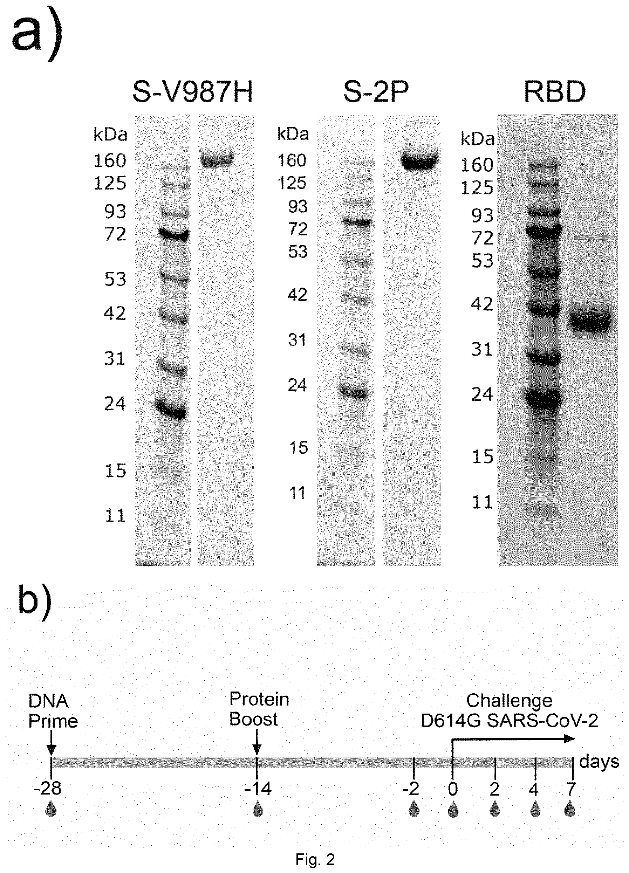

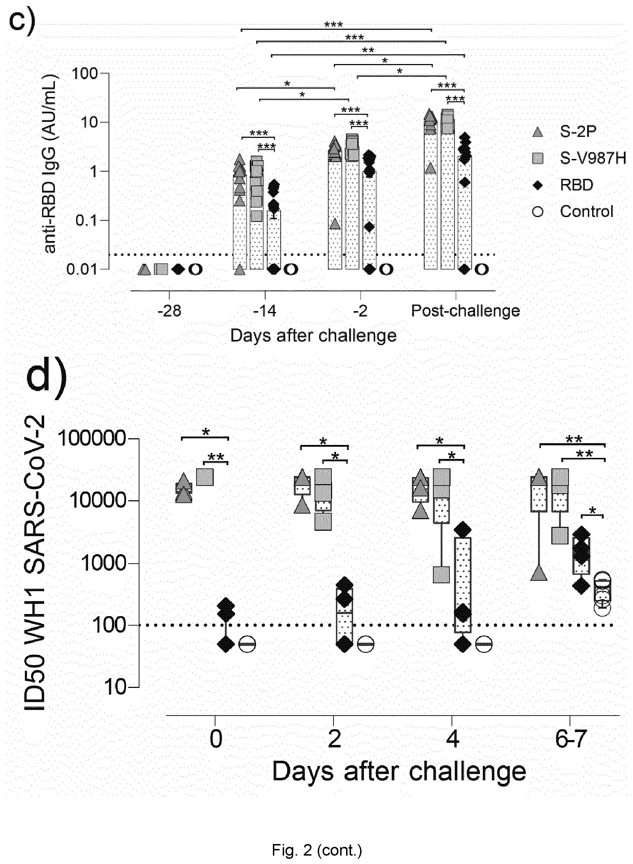

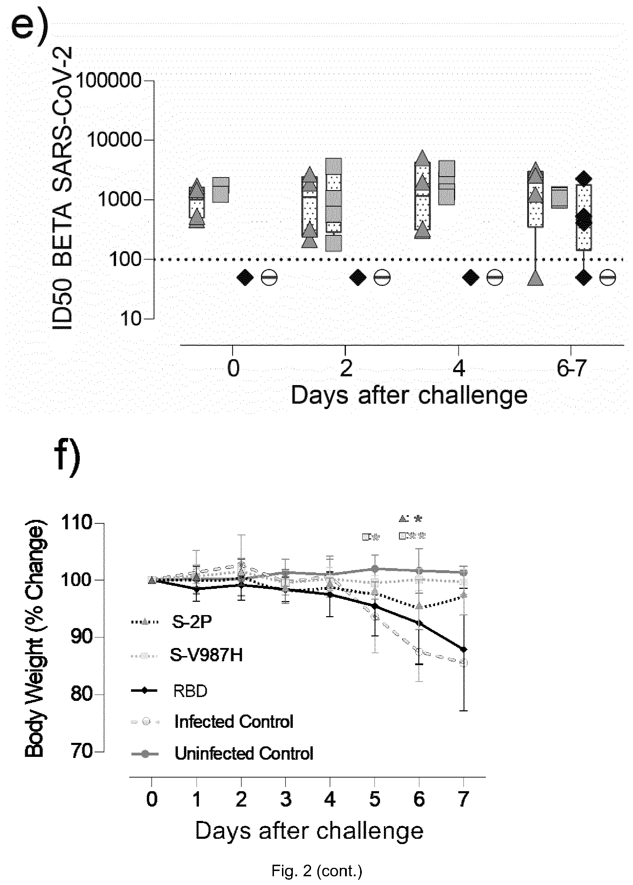

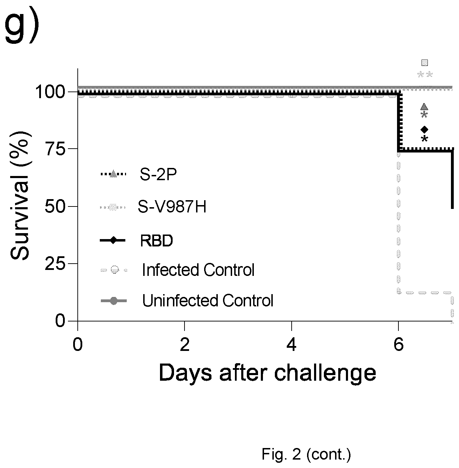

**Figure 2**: Prophylactic activity of S-V987H immunization and analysis of the humoral response elicited in vaccinated K18-hACE2 mice challenged with the SARS-CoV-2 D614G variant. K18-hACE2 mice were immunized following a DNA/protein prime/boost strategy with S-V987H, S-2P or RBD, and challenged with SARS-CoV-2 D614G. The humoral response, weight changes and survival of mice were evaluated after immunization and/or viral challenge. **a)** Purified S-V987H, S-2P and RBD were analyzed by SDS-PAGE and Coomassie G-250 staining. **b)** Overview of vaccination strategy and infection timeline. Blood drops indicate collection of biological samples. **c)** Kinetics of anti-RBD IgG in serum samples express as arbitrary units (AU) per mL. Triangles: S-2P group (n= 16). Squares: S-V987H group (n= 14). Black diamonds: RBD (n=15). White circles: unvaccinated mice (n=19). Groups in each time point were analyzed using Conover Iman test with multiple comparison correction by FDR. Differences among animals within a particular group along time were analyzed using the Friedman test corrected for multiple comparison using FDR. Mean plus standard errors of the means (SEM) are shown. **d)** Levels of NAbs against SARS-CoV-2WH1 variant after viral challenge. **e)** Levels of NAbs against SARS-CoV-2 B.1.351 (Beta) variant after viral challenge. Neutralization data were analyzed as indicated in b. **f)** Percentage of weight variation in SARS-CoV-2 D614G-infected K18-hACE2 mice over time. Statistical analysis was performed comparing each vaccinated group with the unvaccinated group using KrusKal-Wallis corrected by Dunn's test. **g)** Kaplan-Meier plot showing the survival rate during the course of the experiment. Statistical analysis was performed against unvaccinated group using Mantel-Cox test. * *p*<0.05, ** *p*<0.01, *** *p*<0.001.

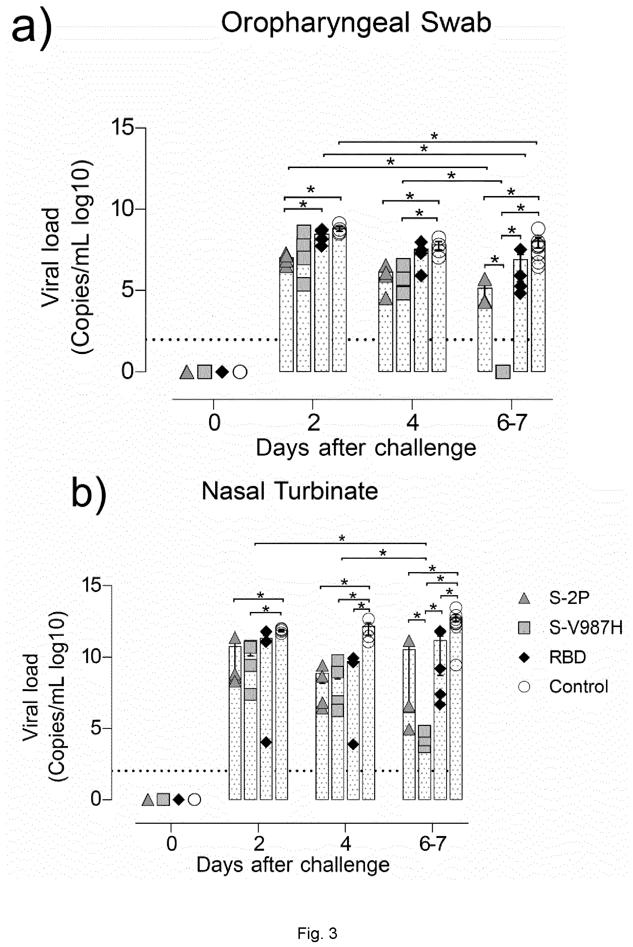

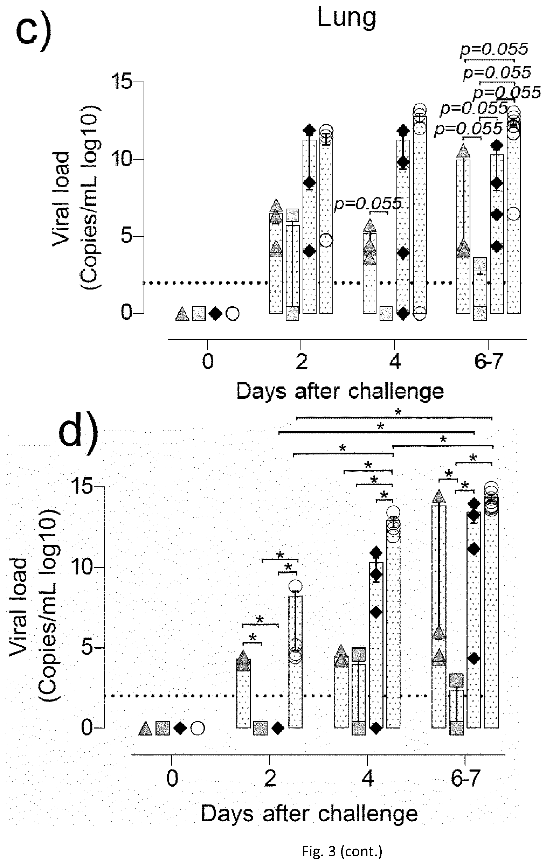

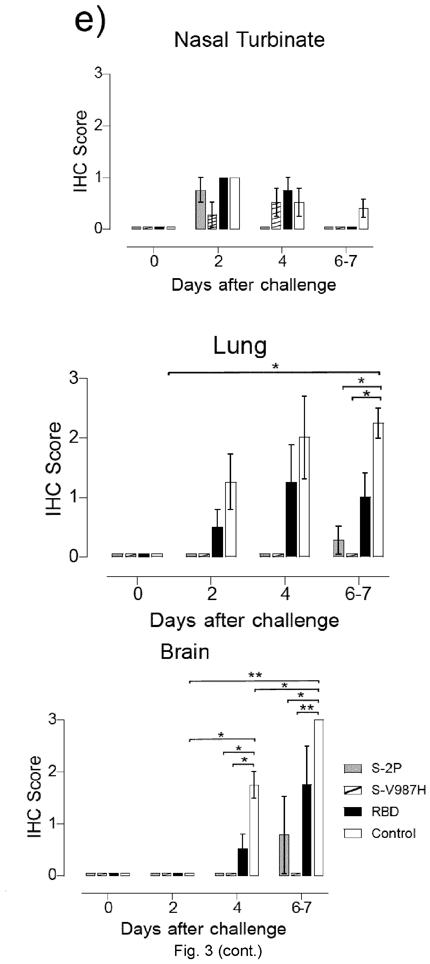

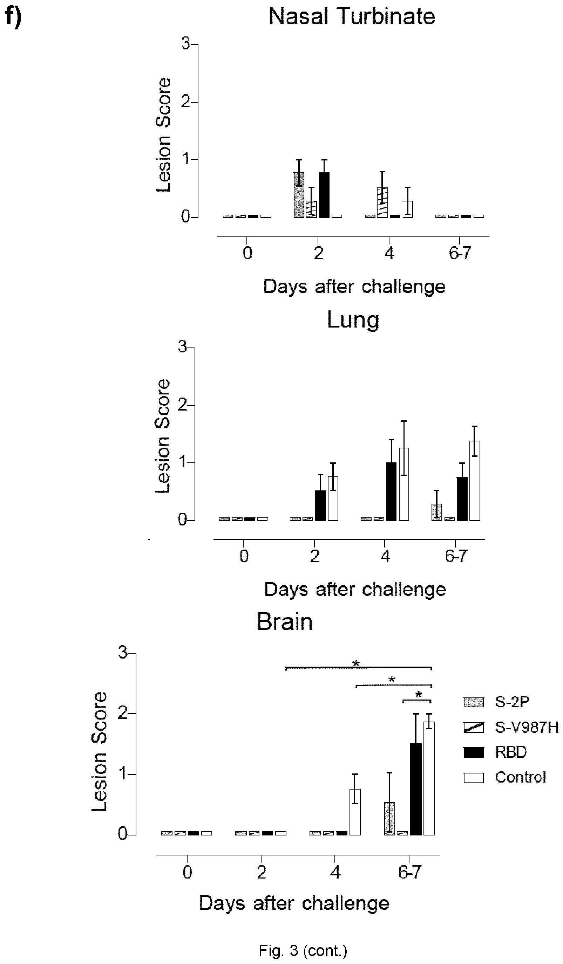

**Figure 3****:** Viral load and histopathological analysis of tissue samples from SARS-CoV-2 D614G infected K18-hACE2 mice after vaccination. SARS-CoV-2 viral load was analyzed in oropharyngeal swabs, and samples from nasal turbinate, lung, and brain of K18-hACE2 mice upon challenge. Virus distribution and tissue damage were analyzed by histopathology. Levels of SARS-CoV-2 gRNA (expressed as logarithmic of copies/mL) in **a)** oropharyngeal swabs, **b)** nasal turbinate, **c)** lung, and **d)** brain during infection. Dot line indicates limit of detection (100 copies/mL). Differences between animals were analyzed using Peto & Peto left-censored k sample test, correcting by FDR. **e)** Detection of SARS-CoV-2 nucleocapsid protein in brain, lung, and nasal turbinate by immunohistochemistry. Staining score: (0) no, (1) low, (2) moderate, and (3) high amount of viral antigen. **f)** Histopathological analysis of nasal turbinate, lung and brain by hematoxylin and eosin staining. Lesion score: (0) no, (1) mild, (2) moderate, and (3) severe lesion. Differences between groups were analyzed using Asymptotic Generalized Pearson Chi-Squared test with FDR correction. * *p*<0.05, ** *p*<0.01. P values proximal to statistical significance are shown as numbers.

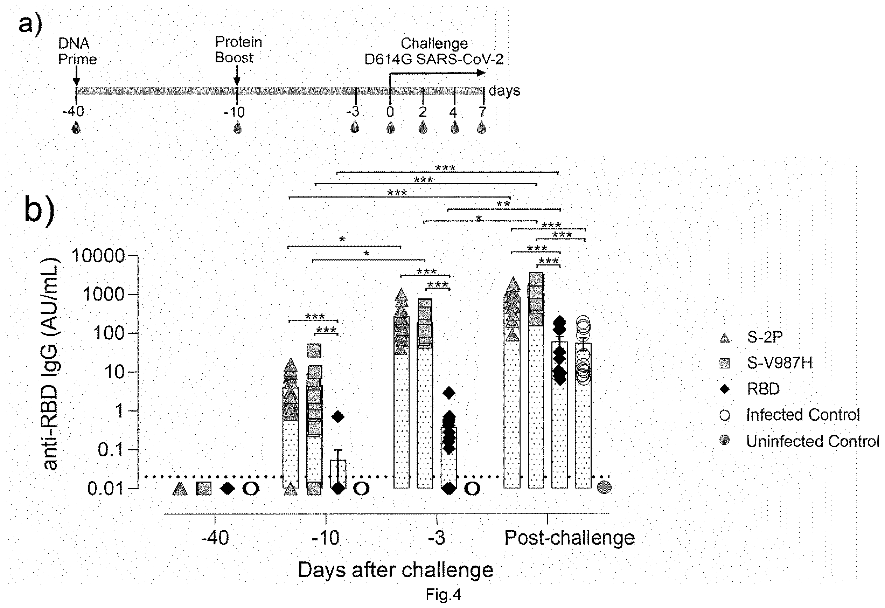

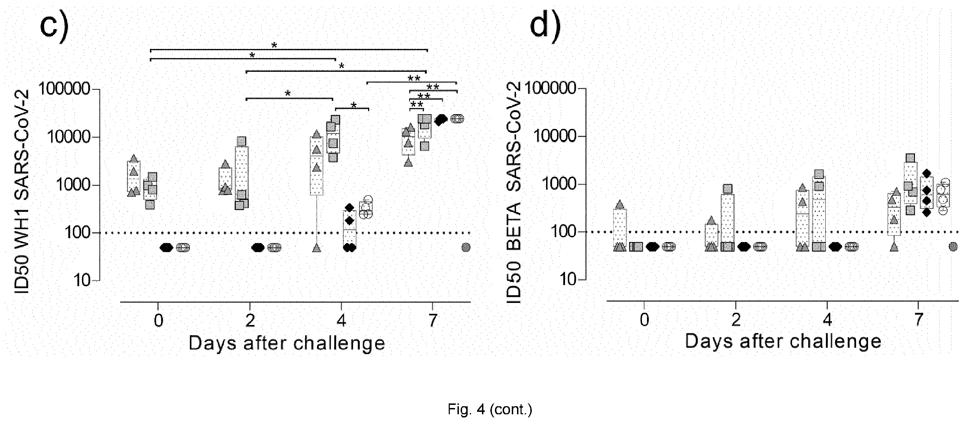

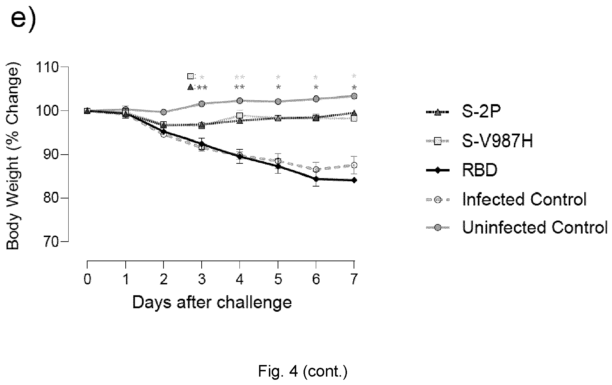

**Figure 4****:** Prophylactic activity of S-V987H immunization and analysis of the humoral response elicited in vaccinated golden Syrian hamsters (GSH) challenged with the SARS-CoV-2 D614G variant. GSH were immunized following a prime/boost strategy with S-V987H, S-2P or RBD, and challenged with SARS-CoV-2 D614G. The humoral response and weight changes of GSH were evaluated after immunization and/or viral challenge. **a)** Outline of vaccination strategy and infection timeline. Blood drops indicate collection of biological samples. **b)** Kinetics of anti-RBD antibodies in serum samples. Triangles: S-2P group (n= 16). Squares: S-V987H group (n= 16). Black diamonds: RBD (n=16). White circles: unvaccinated and challenged GSH (n=16). Grey circles: unvaccinated and uninfected GSH (n=4). Groups in each time point were analyzed using Conover-Iman test with multiple comparison correction by FDR. Differences among animals within a particular group along time were analyzed using the Friedman test with FOR correction. **c)** Sera neutralizing activity against SARS-CoV-2 WH1 and **d)** SARS-CoV-2 B.1.351 (Beta) variants after viral challenge. Neutralization data were analyzed as indicated in a. **e)** Percentage of weight variation in SARS-CoV-2 D614G-infected GSH over time. Statistical analysis was performed against the unvaccinated group using Krustal-Wallis correcting by Duns test. * *p*<0.05, ** *p*<0.01, *** *p*<0.001.

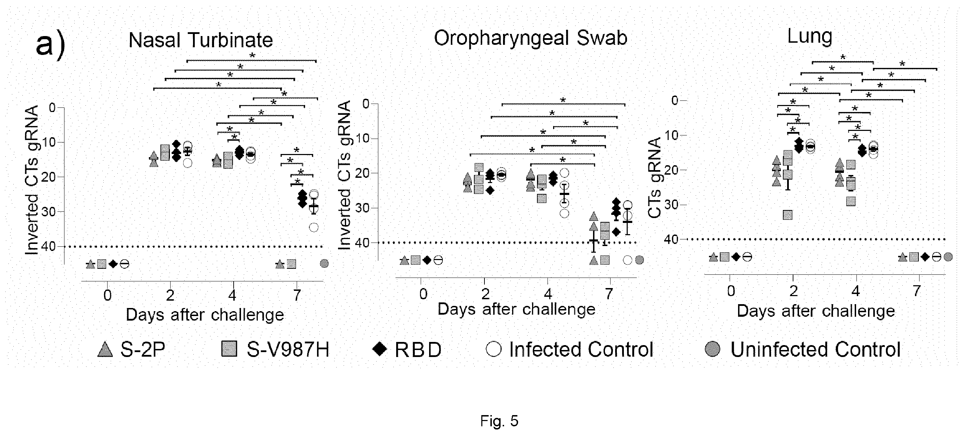

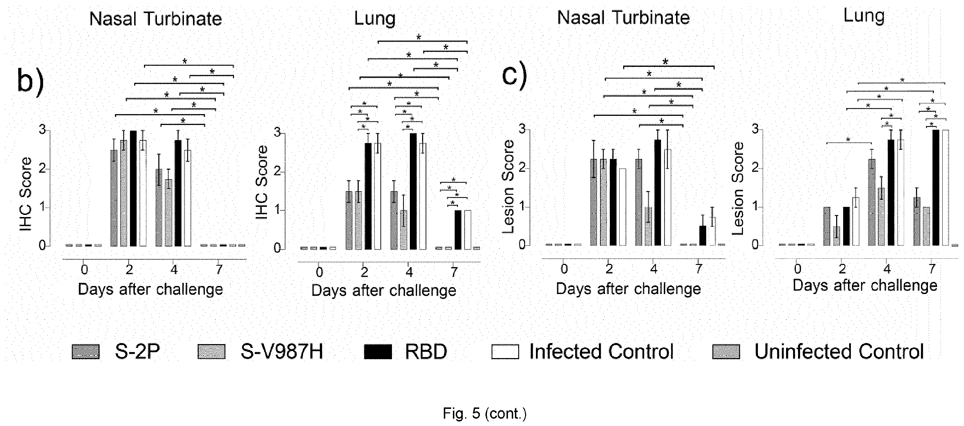

**Figure 5****:** Viral load and histopathology analysis of tissue samples from SARS-CoV-2 D614G infected golden Syrian hamsters after vaccination. SARS-CoV-2 viral load was analyzed in oropharyngeal swabs, and samples from nasal turbinate and lung of infected GSHs. Virus distribution and tissue damage was analyzed by histopathology. **a)** Levels of SARS-CoV-2 gRNA, expressed as cycles threshold (Cts), in oropharyngeal swabs, nasal turbinate and lung during infection. Dot line indicates limit of detection (Cts≥40). Differences between groups were analyzed using Peto & Peto left-censored k sample test with FOR correction. **b)** Detection of SARS-CoV-2 nucleocapsid protein in lung and nasal turbinate by immunohistochemistry. Staining score: (0) no, (1) low, (2) moderate, and (3) high amount of viral antigen. **c)** Histopathologic analysis of nasal turbinate and lung by hematoxylin and eosin staining. Lesion score: (0) no, (1) mild, (2) moderate, and (3) severe lesion. Differences between groups were analyzed by the Asymptotic Generalized Pearson Chi-Squared test corrected using FDR. * *p*<0.05.

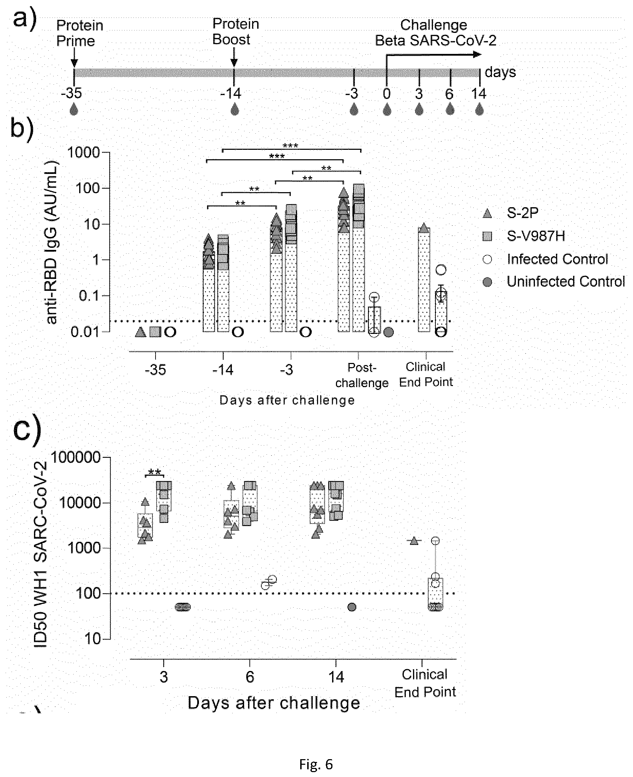

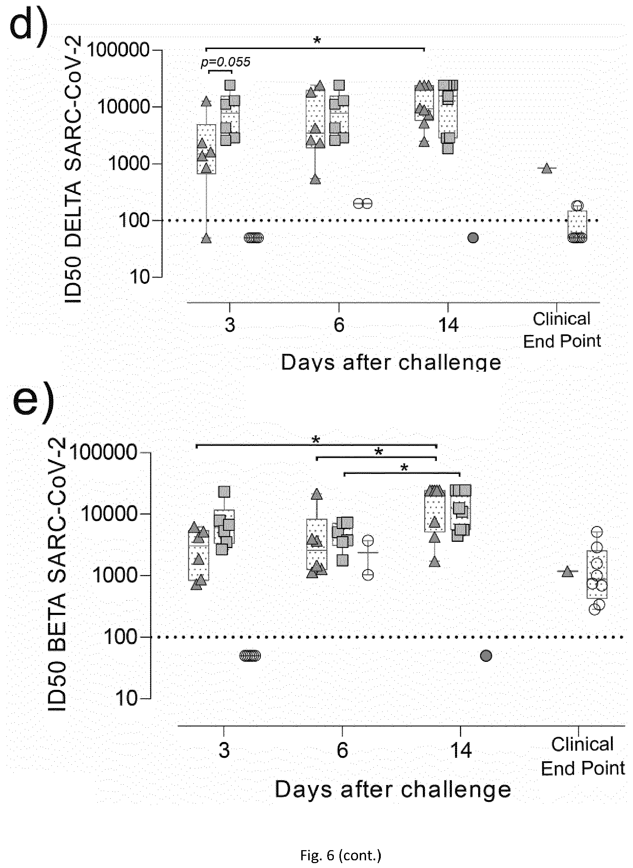

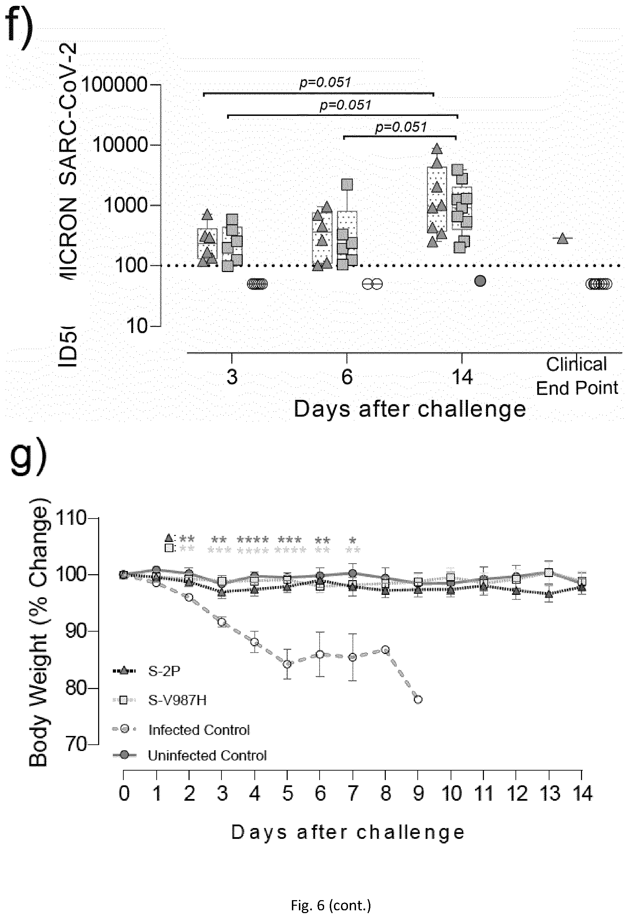

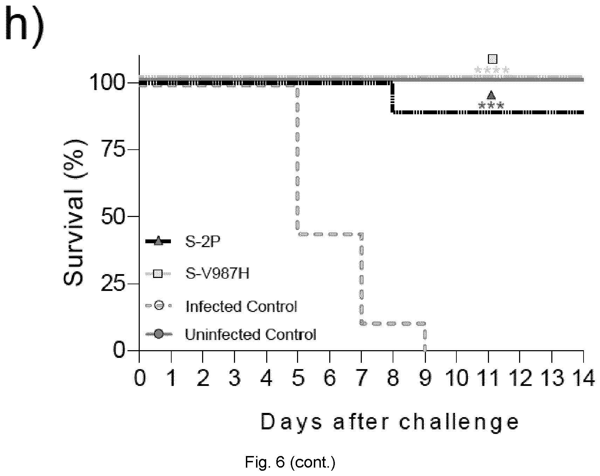

**Figure 6****:** Prophylactic activity of S-V987H immunization and analysis of the humoral response elicited in vaccinated K18-hACE2 mice challenged with the SARS-CoV-2 B.1.351 (Beta) variant. K18-hACE2 mice were immunized twice with S-V987H or S-2P, adjuvanted with AddaVax. Then, mice were challenged with the SARS-CoV-2 B.1.351 Variant of Concern (VoC). The humoral response, weight changes and survival of mice were evaluated after immunization and/or viral challenge. **a)** Overview of vaccine strategy and infection timeline. Blood drops indicate collection of biological samples. **b)** Kinetics of anti-RBD antibodies in serum samples. Triangles: S-2P group (n= 21). Squares: S-V987H group (n= 21). White circles: unvaccinated-challenge mice (n=16). Grey circles: uninfected and unvaccinated mice (n=10). Groups in each time point were analyzed using Conover-Iman test with multiple comparison correction by FDR. Differences among animals within a particular group along time were analyzed using Friedman test with FOR correction. Sera neutralizing activity against: **c)** SARS-CoV-2 WH-1, **d)** B.1.617.2 (Delta), **e)** B.1.351 (Beta), and **f)** B.1.1.529 (Omicron) variants after viral challenge. Neutralization data were analyzed as indicated in a. **g)** Percentage of weight variation in SARS-CoV-2 B.1.351 infected K18-hACE2 mice over time. Statistical analysis was performed against the unvaccinated and challenged group using Kruskal-Wallis with Dunn's test correction. **h)** Kaplan-Meier plot showing the percentage of SARS-CoV-2-infected animals that were survive at the end of the experiment. Statistical analysis was performed against unvaccinated group using Mantel-Cox test. * *p*<0.05, ** *p*<0.01, *** *p*<0.001, **** *p*<0.0001. Mean plus standard errors of the means (SEM) are shown.

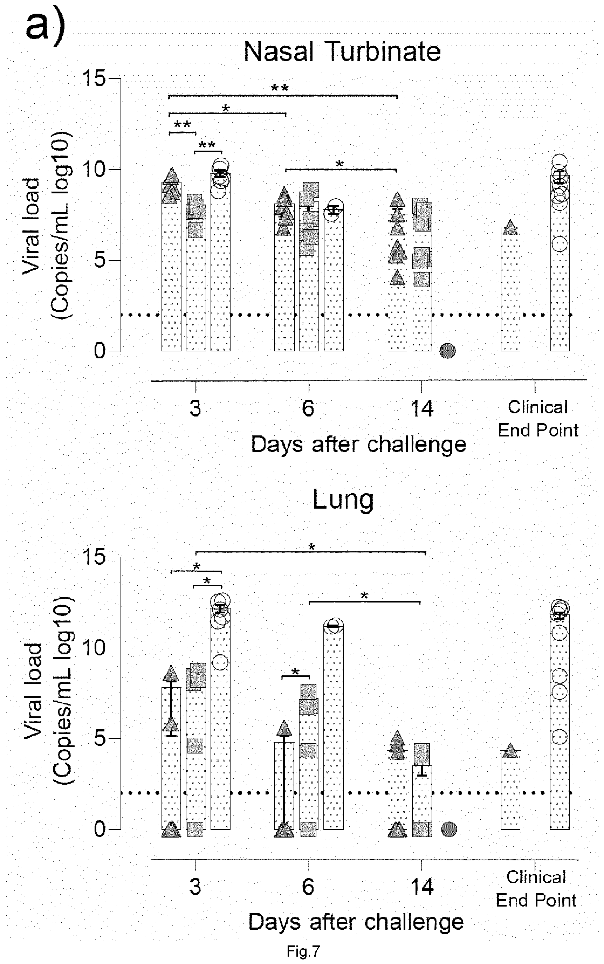

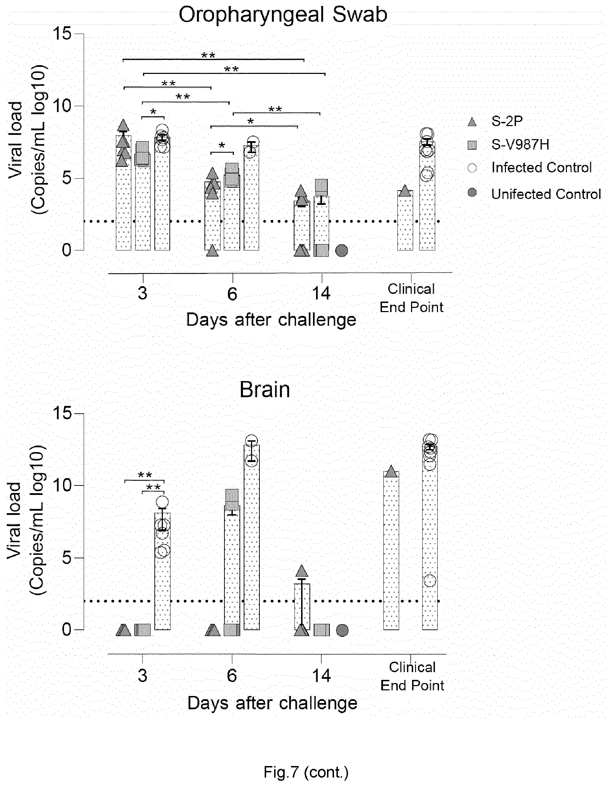

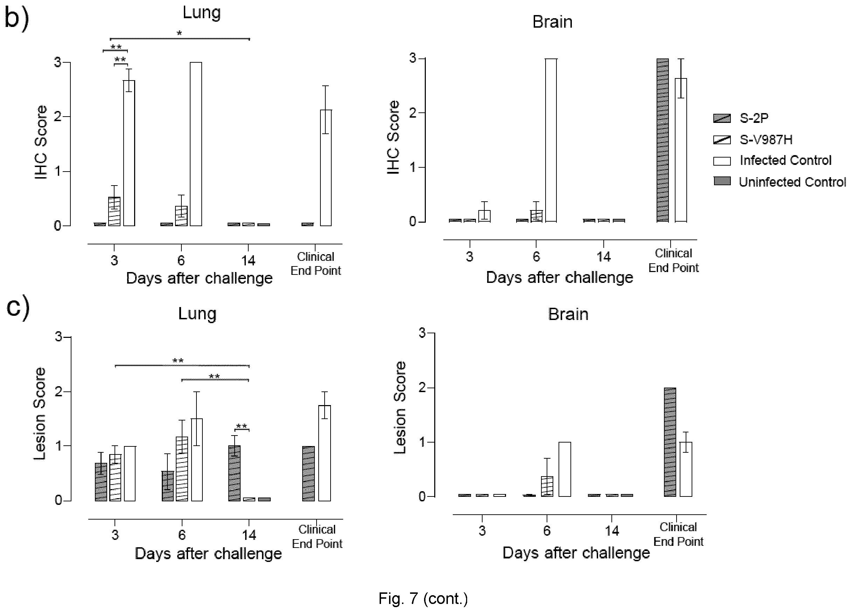

**Figure 7****:** Viral load and histopathology analysis of tissue samples from SARS-CoV-2 B.1.351 infected K18-hACE2 mice after vaccination. SARS-CoV-2 viral load was analyzed in oropharyngeal swabs, and samples from nasal turbinate, lung and brain of infected K18-hACE2. Virus distribution and tissue damage was analyzed by immunohistochemistry and histopathology. **a)** Levels of SARS-CoV-2 gRNA (expressed as logarithmic of copies/mL) in oropharyngeal swabs, nasal turbinate, lung, and brain during infection. Dot line indicates limit of positivity (100 copies/mL). Differences between groups were analyzed using Peto & Peto left-censored k sample test with FOR correction. **b)** Detection of SARS-CoV-2 nucleocapsid protein in brain, lung, and nasal turbinate by immunohistochemistry. Staining score: (0) no, (1) low, (2) moderate, and (3) high amount of viral antigen. **c)** Histopathological analysis of brain, lung, and nasal turbinate by hematoxylin and eosin staining. Lesion score: (0) no, (1) mild, (2) moderate, and (3) severe lesion. Differences between groups were analyzed using Asymptotic Generalized Pearson Chi-Squared test with FDR correction. * p<0.05, ** p<0.01.

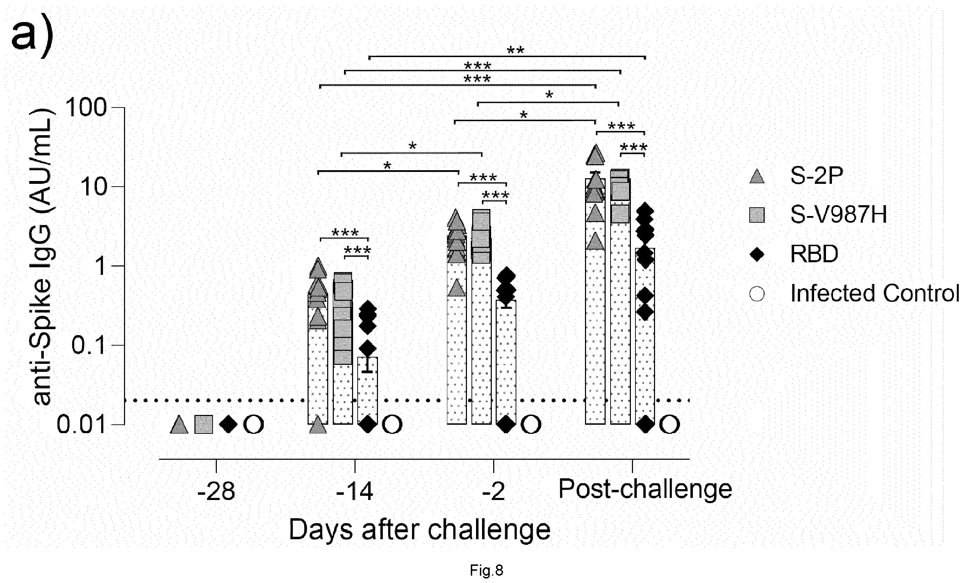

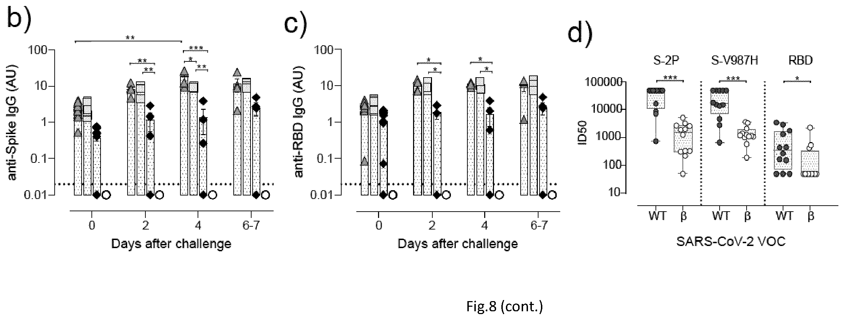

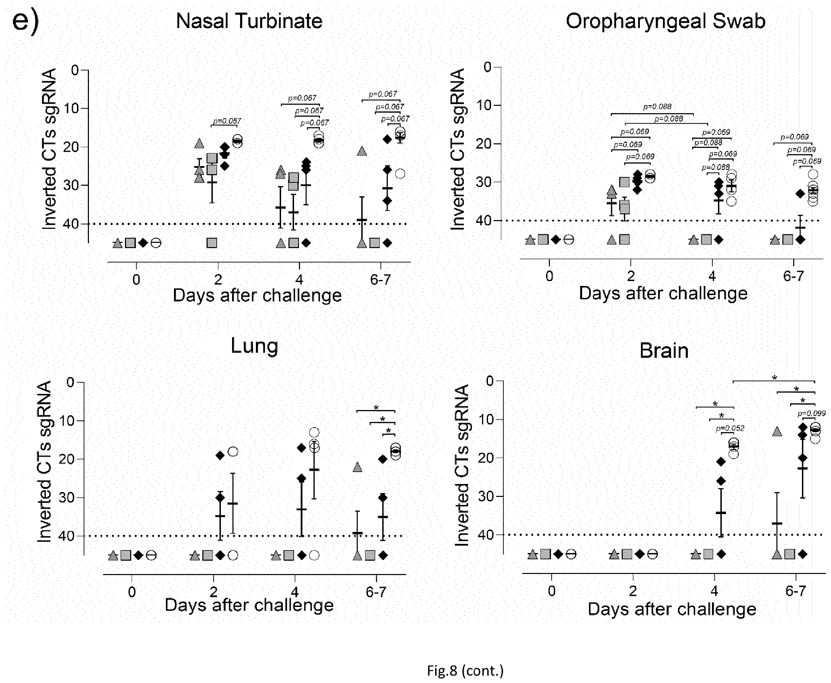

**Figure 8****:** Analysis of anti-Spike IgG responses and levels of tissue sub-genomic RNA in immunized K18-hACE2 challenged with SARS-CoV-2 D614G. **a)** Kinetics of anti-Spike IgG antibodies in serum samples. Triangles: S-2P group (n= 16). Squares: S-V987H group (n= 14). Black diamonds: RBD (n=15). White circles: unvaccinated mice (n=19). Groups in each time point were analyzed using Conover-Iman test with multiple comparison correction by FDR. Differences among animals within a particular group along time were analyzed using the Friedman test corrected for multiple comparison using FDR. **b)** & **c)** Kinetics of anti-Spike and anti-RBD IgG antibodies respectively, in serum samples on days 0, 2, 4 and 6-7 (end point) after SARS-CoV-2 D614G challenge. Mean plus standard error of the mean (SEM) is shown. **d)** NAbs titers against SARS-CoV-2 WH-1 in purple and SARS-CoV-2 B.1.351 (Beta) variant in green, after viral challenge. **e)** Levels of SARS-CoV-2 subgenomic RNA (represented as inverted Ct) in oropharyngeal swabs, nasal turbinate, lung, and brain after virus challenge. Dot line indicates limit of detection (Ct=40). Difference in neutralizing titers were analyzed using Wilcoxon rank sum test. Differences between groups for humoral response and sgRNA were analyzed using Peto & Peto left-censored k sample test corrected by FDR. * *p*<0.05, ** *p*<0.01, *** *p*<0.001 P values proximal to statistical significance are shown as numbers.

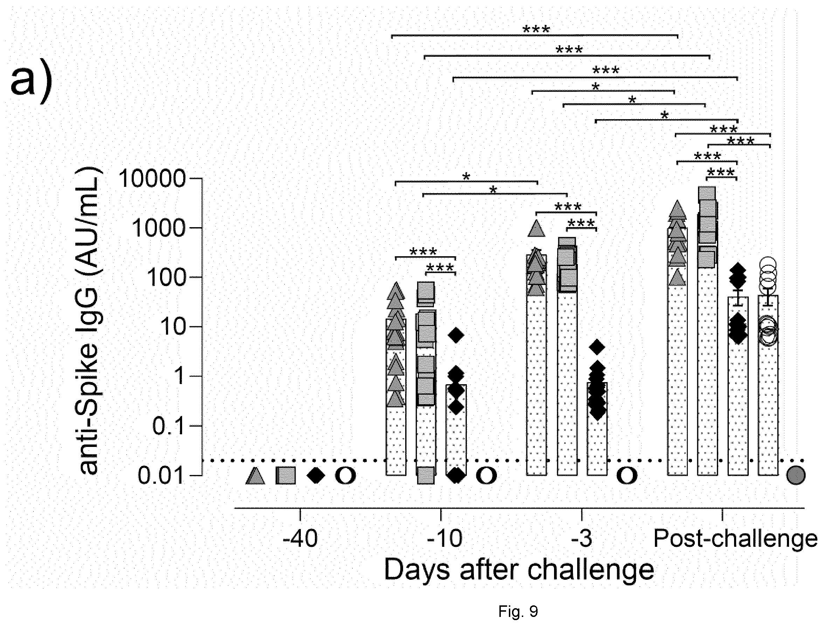

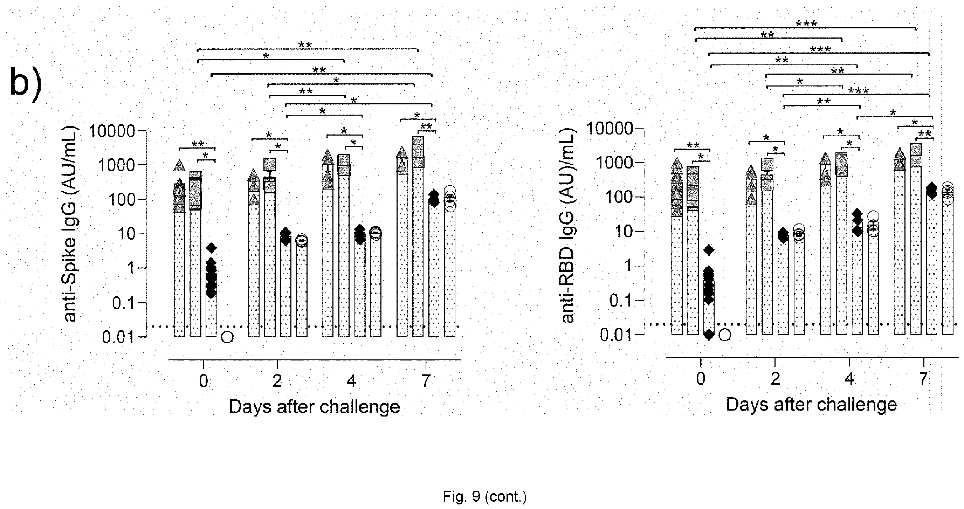

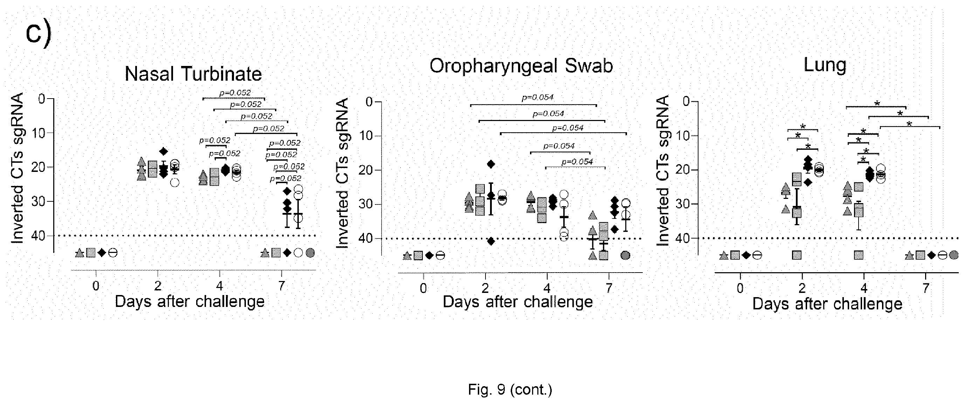

**Figure 9****:** Analysis of anti-Spike IgG responses and levels of tissue sub-genomic RNA in immunized golden Syrian hamsters challenged with SARS-CoV-2 D614G. **a)** Kinetics of anti-Spike antibodies in serum samples. Triangles: S-2P group (n= 16). Squares: S-V987H group (n= 16). Black diamonds: RBD (n=16). White circles: unvaccinated and infected GSH (n=16). Grey circles: unvaccinated and unchallenged GSH (n=4). Groups in each time point were analyzed using Conover-Iman test with multiple comparison correction by FDR. Differences among animals within a particular group along time were analyzed using the Friedman test corrected for multiple comparison using FDR. **b)** Kinetics of anti-Spike and anti-RBD IgG antibodies in serum samples on days 0, 2, 4 and 6-7 (end point) after SARS-CoV-2 D614G challenge. Mean plus standard error of the mean (SEM) are shown. **c)** Levels of SARS-CoV-2 subgenomic RNA (represented as inverted Ct) in oropharyngeal swabs, nasal turbinate and lung after virus challenge. Dot line indicates limit of detection (40 Cts). Differences between groups were analyzed using Peto & Peto left-censored k sample test corrected by FDR. * *p*<0.05, ** *p*<0.01, *** *p*<0.001. P values proximal to statistical significance are shown as numbers.

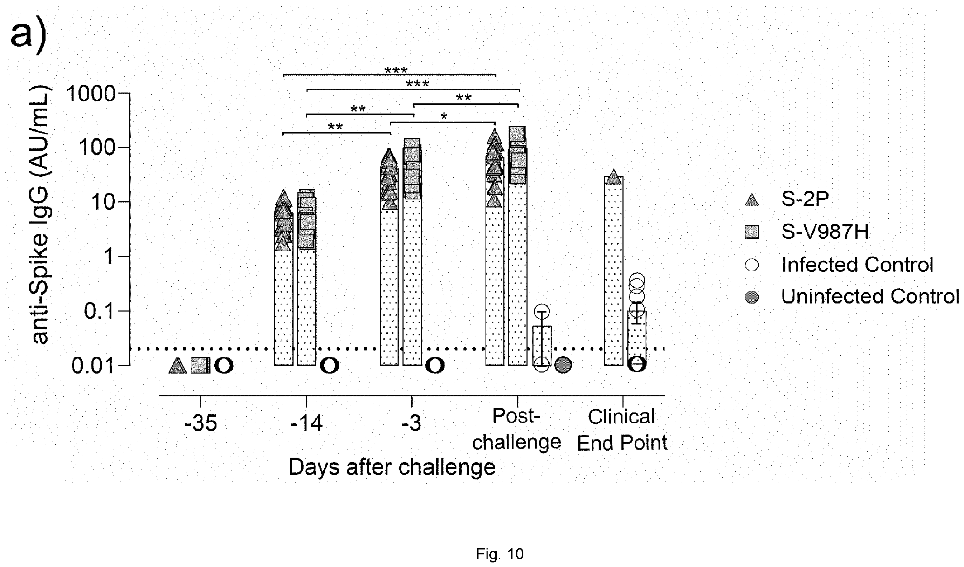

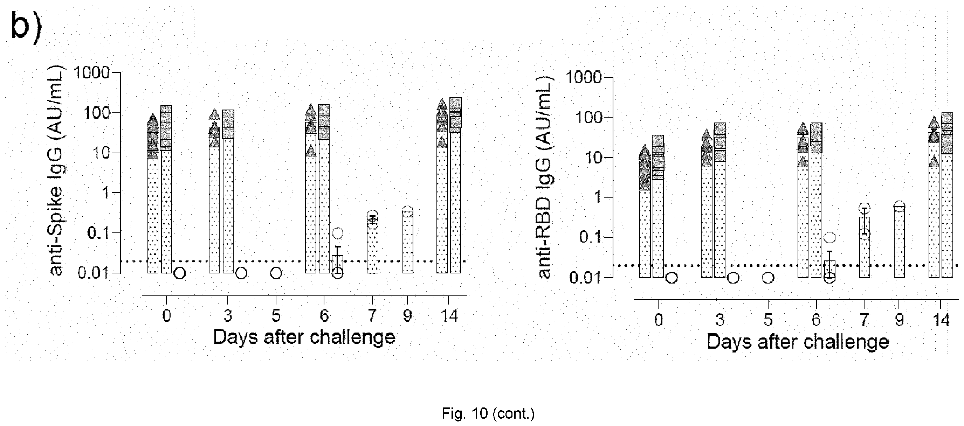

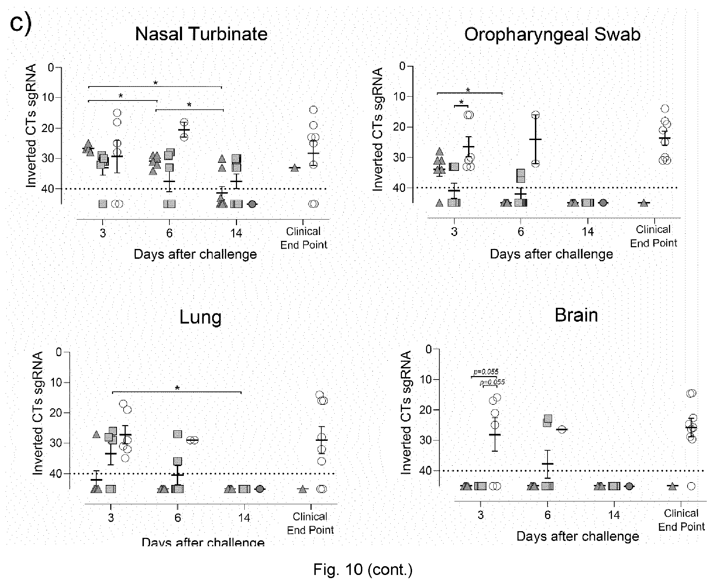

**Figure 10****:** Analysis of anti-Spike IgG responses and levels of tissue sub-genomic RNA in immunized K18-hACE2 challenged with SARS-CoV-2 B.1.351 (Beta) variant. **a)** Kinetics of anti-Spike IgG antibodies in serum samples. Triangles: S-2P group (n= 21). Squares: S-V987H group (n= 21). White circles: unvaccinated-challenge mice (n=16). Grey circles: uninfected and unvaccinated mice (n=10). Groups in each time point were analyzed using Conover-Iman test with multiple comparison correction by FDR. Differences among animals within a particular group along time were analyzed using the Friedman test corrected for multiple comparison using FDR. **b)** Kinetics of anti-Spike and anti-RBD IgG antibodies in serum samples on days 0, 2, 4 and 6-7 (end point) after SARS-CoV-2 B.1.351 challenge. Mean plus standard error of the mean (SEM) is shown. **c)** Levels of SARS-CoV-2 subgenomic RNA (represented as inverted Ct) in oropharyngeal swabs, nasal turbinate, lung, and brain after virus challenge. Dot line indicates limit of detection (40 Cts). Differences between groups were analyzed using Peto & Peto left-censored k sample test corrected by FDR. * *p*<0.05, ** *p*<0.01, *** *p*<0.001.

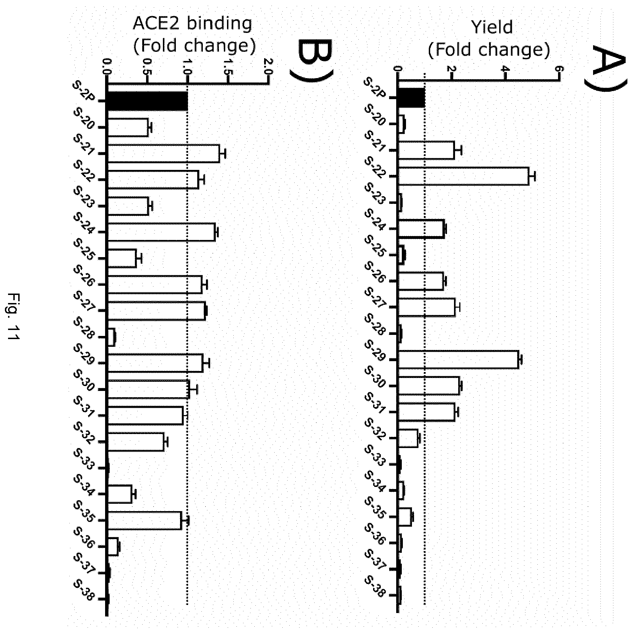

**Figure 11****:** Yield and RBD exposure of stabilized Spike glycoproteins. **a)** Yields of recombinant Spike variants in a five-day cell culture supernatant. Mean plus standard deviation of three experiments are shown. **b)** RBD exposure index in selected recombinant proteins. Data are shown as ratio between RBD binding and total protein. Mean plus standard deviation of three experiments are shown.

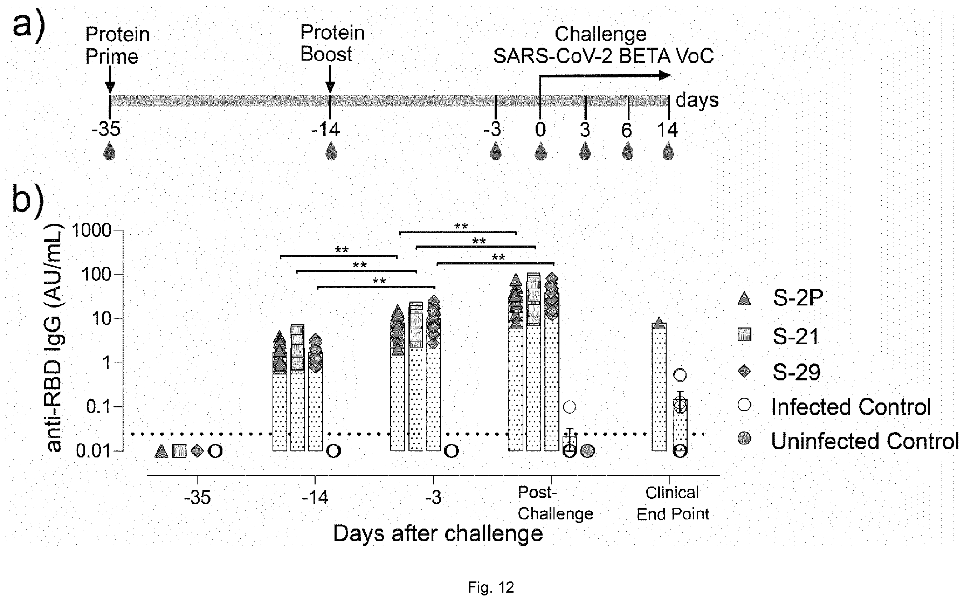

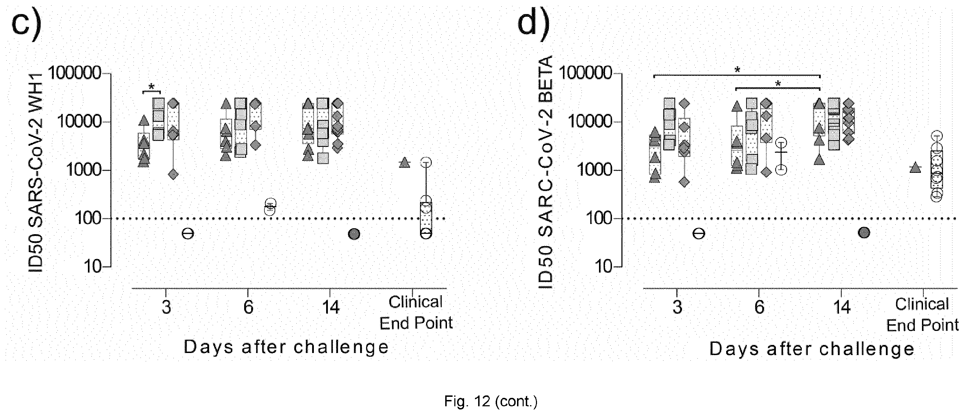

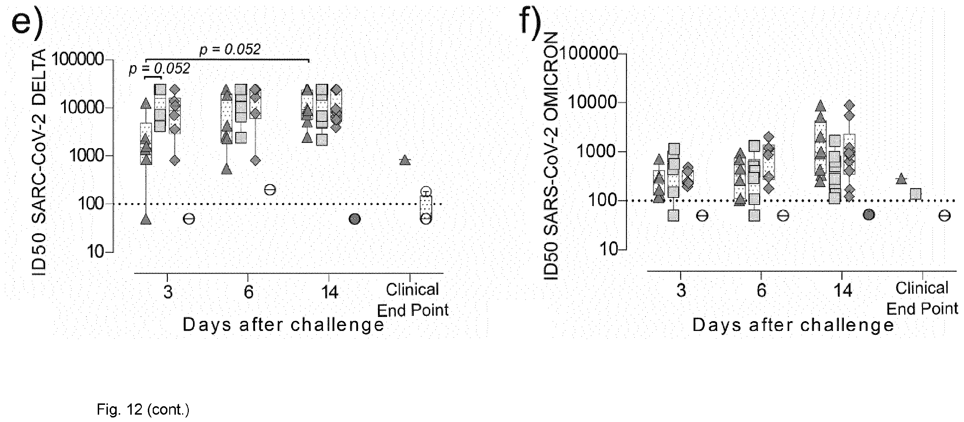

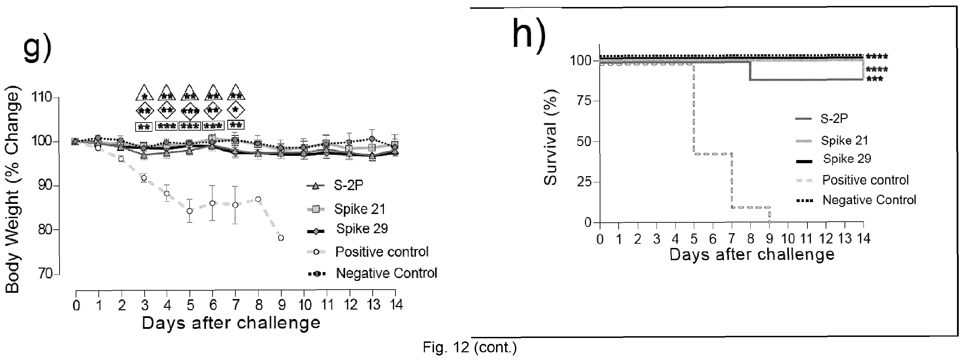

**Figure 12****:** Prophylactic activity of S-21 and S-29 immunization and vaccine-induced humoral response elicited in K18-hACE2 mice challenged with SARS-CoV-2 B.1.351 (Beta) variant. K18-hACE2 mice were immunized following a prime/boost strategy with S-21, S-29, or S-2P, and challenged with SARS-CoV-2 Beta. The vaccine-induced humoral responses, weight changes, and survival of mice were evaluated after immunization and/or viral challenge. **a)** Overview of immunization strategy and infection timeline. Blood drips indicate collection of biological samples. **b)** Kinetics of anti-RBD antibodies in serum samples. Triangles: S-2P group (n=21). Squares: S-21 (n=22). Diamond: S-29 (n=22). White circles: unvaccinated-challenge mice (n=16). Grey circles: unvaccinated-uninfected mice (n=10). Groups in each time point were analyzed using Connover Test with multiple comparison correction by FDR. Differences among animals within a particular group along time were analyzed using Friedman test with FOR correction. Sera neutralizing activity against: **c)** SARS-CoV-2 WH-1 variant, **d)** B.1.351 (Beta) variant, **e)** B.1.617.2 (Delta), and **f)** B.1.1.529 (Omicron) VoCs after viral challenge. Neutralization data were analyzed as indicated in b. **g)** Percentage of weight variation in SARS-CoV-2 B.1.351 infected K18-hACE2 mice over time. Statistical analysis was performed against the unvaccinated and challenged group using Krustal Wallis with Dunn's test correction. **h)** Kaplan-Meier plot showing the percentage of SARS-CoV-2-infected animals that were disease-free at the end of the experiment. Statistical analysis was performed against unvaccinated group using Mantel-Cox test. * *p*<0.05, ** *p*<0.01, *** *p*<0.001, **** *p*<0.0001. Mean plus standard errors of the means (SEM) are shown.

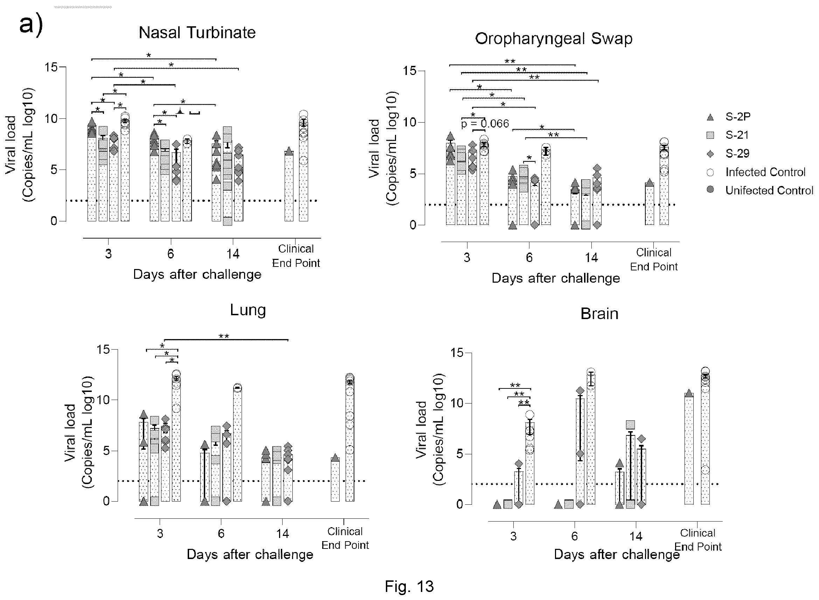

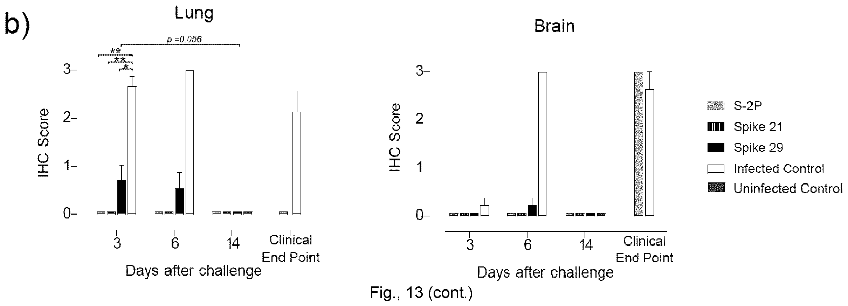

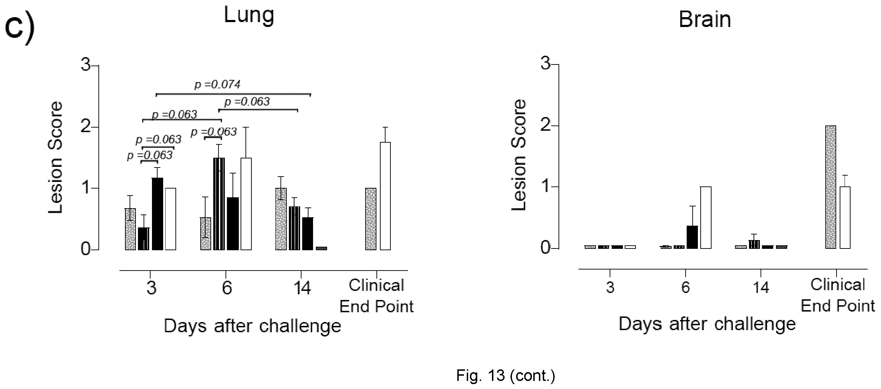

**Figure 13****:** Viral load and histopathology analysis of biological samples from SARS-CoV-2 B.1.351 infected K18-hACE2 mice after vaccination. SARS-CoV-2 viral loads were analyzed in oropharyngeal swabs, and samples from nasal turbinate, lung, and brain of infected K18-hACE2 mice. Virus distribution and tissue damage was analyzed by histopathology. **a)** Levels of SARS-CoV-2 gRNA (expressed as logarithmic of copies/mL) in oropharyngeal swabs, nasal turbinate, lung, and brain during infection. Dotted line indicates limit of detection (100 copies/mL). Differences among groups were analyzed using Peto & Peto left-censored k sample test with FOR correction. **b)** Histopathology analysis of brain, lung, and nasal turbinate by hematoxylin and eosin staining. Lesion score: (0) no lesion, (1) mild lesion, (2) moderate lesion, and (3) severe lesion. **c)** Detection of SARS-CoV-2 nucleocapsid protein in brain, lung, and nasal turbinate by immunohistochemistry. Staining score: (0) no antigen, (1) low, (2) moderate, and (3) high viral antigen. Differences among groups were analyzed using Asymptotic Generalized Pearson Chi-Squared test with FDR correction. * p<0.05, ** p<0.01.

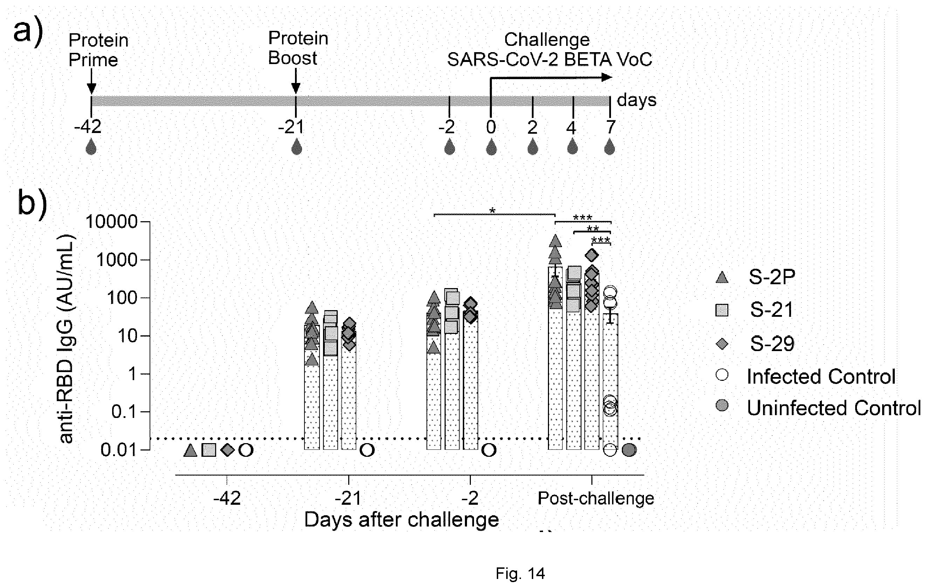

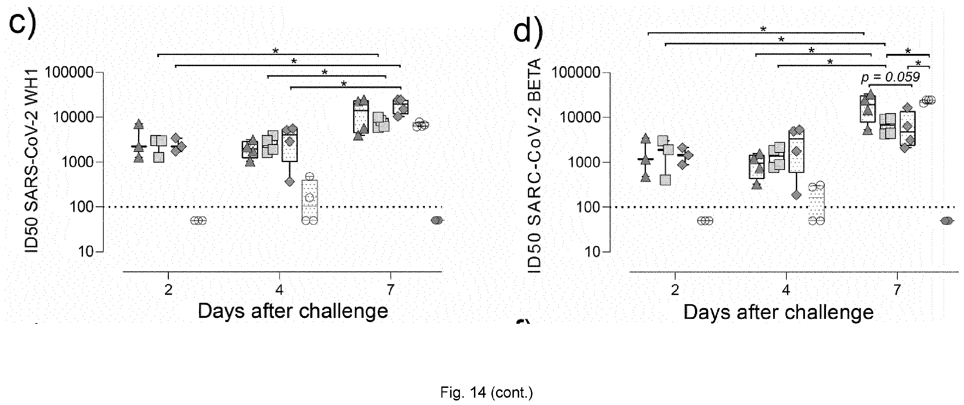

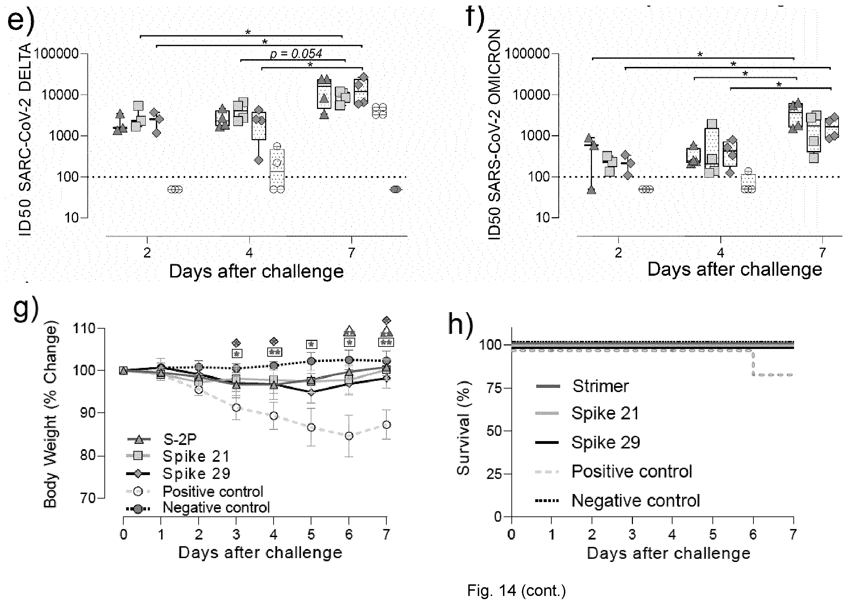

**Figure 14****:** Vaccine-induced humoral responses and prophylactic activity of S-21 and S-29 in immunized GSH after challenge with the SARS-CoV-2 B.1.351 (Beta) variant. GSH were immunized twice with S-21, S-29 or S-2P, and subsequently challenged with SARS-CoV-2 B.1.351 VoC. The humoral response, weight changes, and survival of mice were evaluated after immunization and/or viral challenge. **a)** Outline of immunization schedule and infection timeline. Blood drops indicate collection of biological samples. **b)** Kinetics of anti-RBD antibodies in serum samples. Triangles: S-2P group (n= 11). Squares: S-21 (n=11). Diamond: S-29 (n=11). White circles: unvaccinated-challenge mice (n=11). Grey circles: unvaccinated-uninfected mice (n=5). Groups in each time point were analyzed using Connover Test with multiple comparison correction by FDR. Differences among animals within a particular group along time were analyzed using the Friedman test with FOR correction. Sera neutralizing activity after viral challenge against: **c)** SARS-CoV-2 WH-1, **d)** B.1.351 (Beta), **e)** B.1.617.2 (Delta), and **f)** B.1.1.529 (Omicron) VoCs. Neutralization data were analyzed as indicated in b. **g)** Percentage of weight variation in SARS-CoV-2 B.1.351-infected GSH over time. **h)** Kaplan-Meier plot showing the frequency of disease-free SARS-CoV-2-infected animals at the end of the experiment. Statistical analysis was performed against the unvaccinated group using Krustal Wallis test with multiple comparison correction by Dunn's test. * p<0.05, ** p<0.01, *** p<0.001.

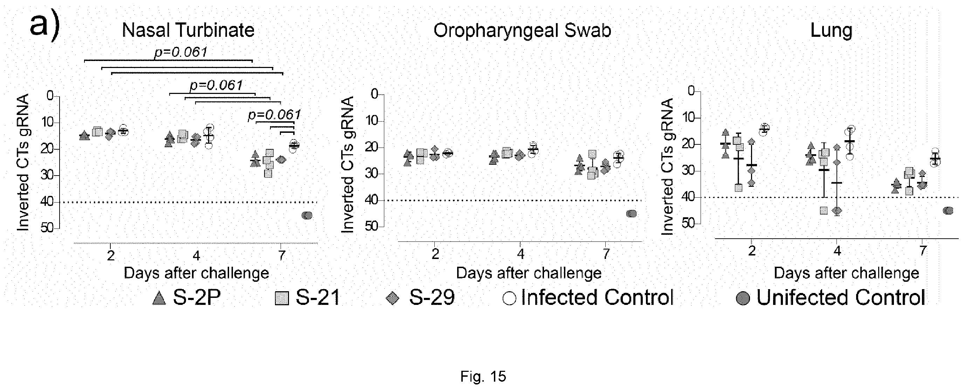

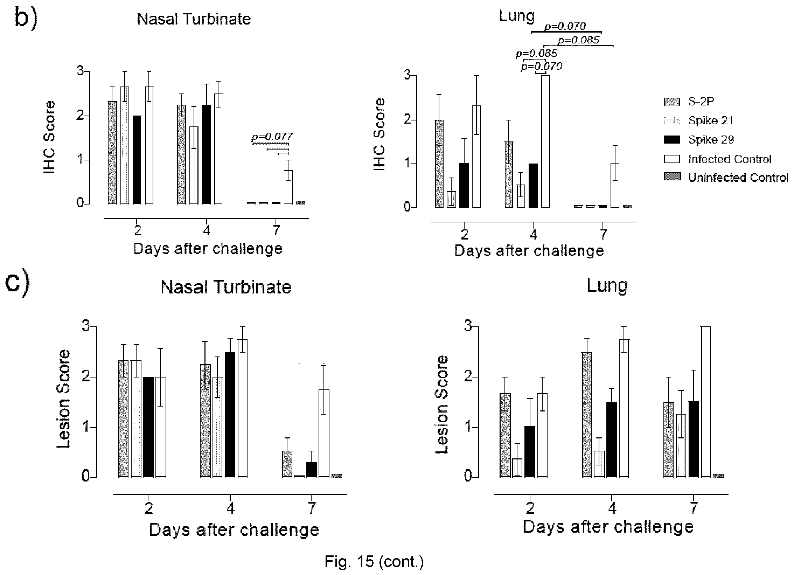

**Figure 15****:** Histopathology and viral loads in tissues from vaccinated GSH after challenge with SARS-CoV-2 Beta VoC. SARS-CoV-2 viral loads were analyzed in oropharyngeal swabs, and samples from nasal turbinate and lung of vaccinated and challenged GSH. Virus distribution and tissue damage was analyzed by histopathology. a) Levels of SARS-CoV-2 gRNA, expressed as cycles threshold (CTs), in oropharyngeal swabs, nasal turbinate, and lung during infection. Dotted line indicates limit of positivity (40 CTs). Differences among groups were analyzed using Peto & Peto left-censored k sample test with FOR correction. **b)** Histopathology analysis of lung and nasal turbinate by hematoxylin and eosin staining. Lesion score: (0) no lesion, (1) mild lesion, (2) moderate lesion, and (3) severe lesion. **c)** Detection of SARS-CoV-2 Nucleocapsid protein in lung and nasal turbinate by IHC. Staining score: (0) no antigen, (1) low, (2) moderate, and (3) high viral antigen. Differences among groups were analyzed using Asymptotic Generalized Pearson Chi-Squared test and FDR.

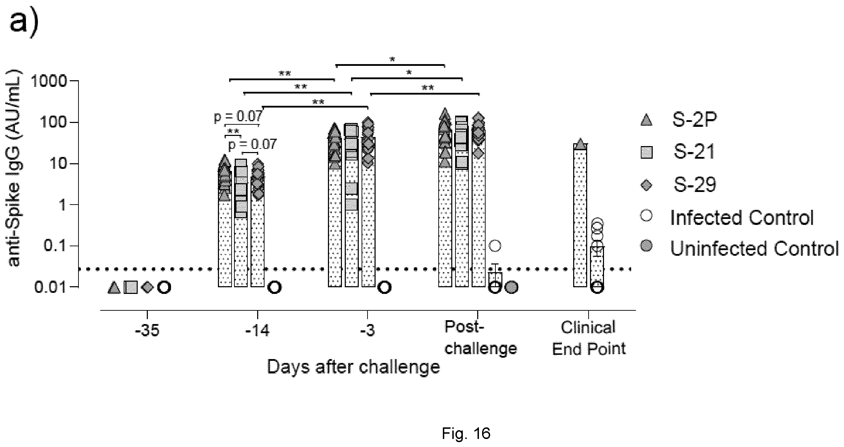

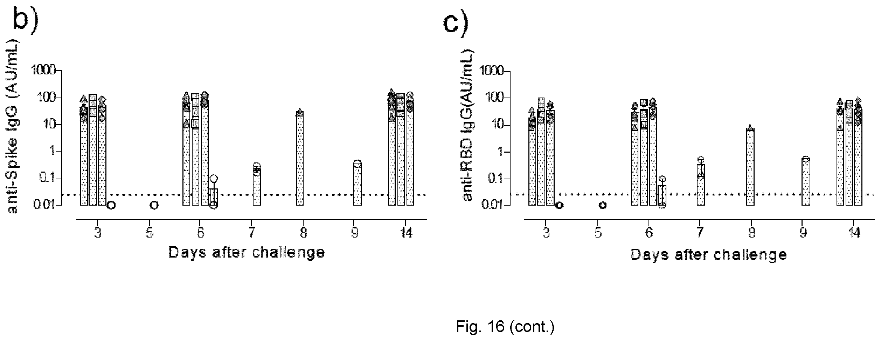

**Figure 16****:** Vaccine-induced anti-Spike IgG responses and levels of tissue sub-genomic RNA in immunized K18-huACE2 mice after challenge with SARS-CoV-2 B.1.351 (Beta) VoC. **a)** Kinetics of anti-Spike IgG antibodies in serum samples. Triangles: S-2P group (n=21). Squares: S-21 (n=22). Diamond: S-29 (n=22). White circles: unvaccinated-challenge mice (n=16). Grey circles: unvaccinated-uninfected mice (n=10). Groups in each time point were analyzed using Connover Test with multiple comparison correction by FDR. Differences among animals within a particular group along time were analyzed using the Friendman test corrected for multiple comparison using FDR. **b)** Kinetics of anti-Spike and anti-RBD IgG antibodies in serum samples after SARS-CoV-2 B.1.351 challenge. Mean plus standard error of the mean (SEM) is shown. **c)** Levels of SARS-CoV-2 subgenomic RNA (represented as inverted Ct) in oropharyngeal swabs, nasal turbinate, lung, and brain after virus challenge. Dotted line indicates limit of detection (40 Cts). Differences among groups were analyzed using Peto & Peto left-censored k sample test corrected by FDR. * p<0.05, ** p<0.01,

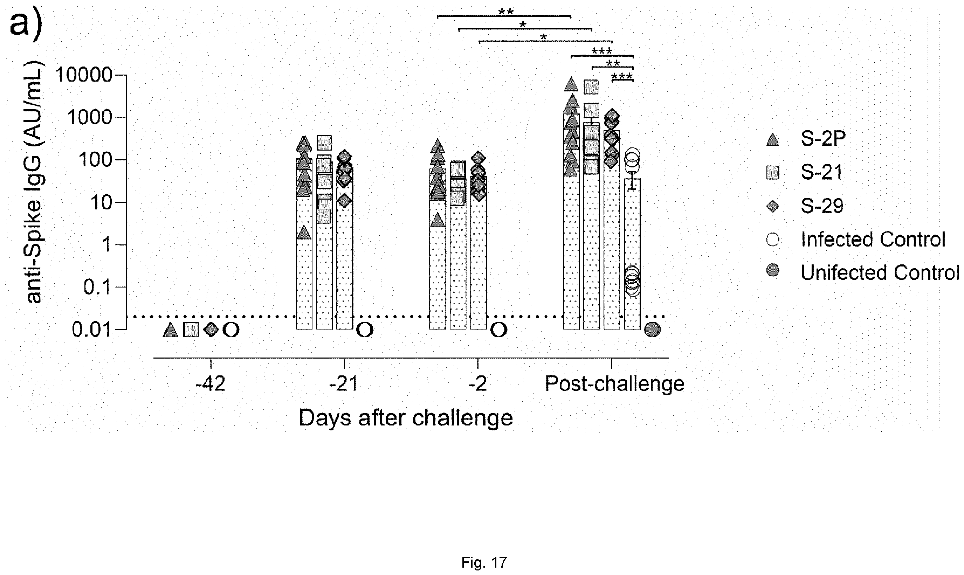

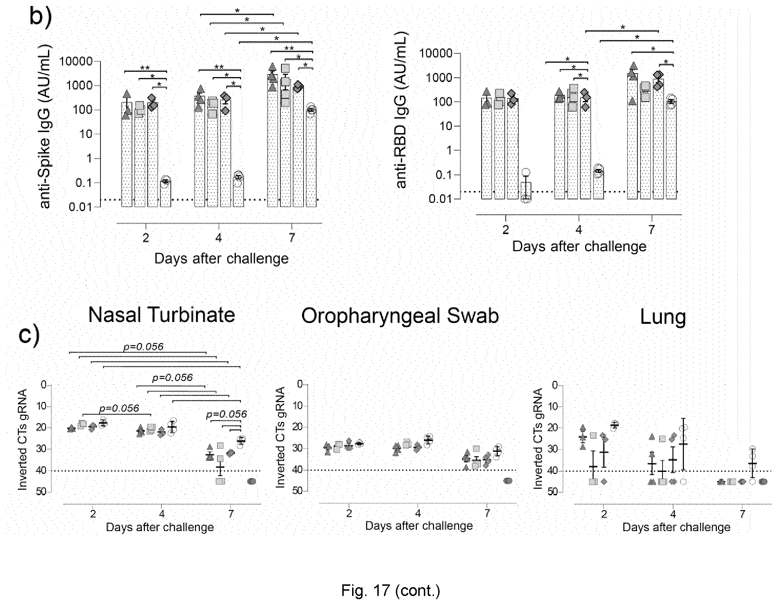

**Figure 17****:** Anti-Spike IgG responses and levels of sub-genomic RNA in tissues of immunized GSH after challenge with SARS-CoV-2 B.1.351 (Beta) variant. a) Kinetics of anti-Spike antibodies in serum samples. Triangles: S-2P group (n= 11). Squares: S-21 (n=11). Diamond: S-29 (n=11). White circles: unvaccinated-challenge mice (n=11). Grey circles: unvaccinated-uninfected mice (n=5). Groups in each time point were analyzed using Connover Test with multiple comparison correction by FDR. Differences among animals within a particular group along time were analyzed using the Friendman test corrected for multiple comparison using FDR. **b)** Kinetics of anti-Spike and anti-RBD IgG antibodies in serum samples at days 2, 4 and 6-7 (end point) after SARS-CoV-2 Beta challenge. Mean plus standard error of the means (SEM) are shown. **c)** Levels of SARS-CoV-2 subgenomic RNA (represented as inverted Ct) in oropharyngeal swabs, nasal turbinate, and lung after virus challenge. Dotted line indicates limit of positivity (40 Cts). Differences among groups were analyzed using Peto & Peto left-censored k sample test corrected by FDR. * p<0.05, ** p<0.01, *** p<0.001.

### DETAILED DESCRIPTION OF THE INVENTION

The present invention relates to the provision of new polypeptides and polynucleotides for increasing stability and production of SARS-CoV-2 spike protein.

### Polypeptide of the SARS-CoV-2 spike protein:

The authors of the present invention have developed a set of variants of the SARS-CoV-2 spike protein which are characterized in that they show increased stability or in that they result in an increased production yield when expressed in a recombinant expression system.

Accordingly, in a first aspect, the invention relates to a polypeptide comprising a region from a SARS-CoV-2 spike protein containing at least one mutation selected from the group consisting of: V987H, L849K, S758E, K947R, Q755R, T961E, V963E, D985L, S982F, L517Y, G416R, K386R, V987W, D427I, D420R, A372W and D420Y, relative to the SARS-CoV-2 spike protein sequence as defined in SEQ ID NO: 77, or a functionally equivalent or immunogenically active variant thereof.

The terms "polypeptide" and "protein" are used interchangeably. The term "protein" refers to a sequence of amino acids composed of the naturally occurring amino acids as well as derivatives thereof. The naturally occurring amino acids are well known in the art and are described in standard text books of biochemistry. Within the amino acid sequence, the amino acids are connected by peptide bonds. Further, the two ends of the amino acid sequence are referred to as the carboxyl terminus (C-terminus) and the amino terminus (N-terminus). The term "protein" encompasses essentially purified proteins or protein preparations comprising other proteins in addition. Further, the term also relates to protein fragments. Moreover, it includes chemically modified proteins. Such modifications may be artificial modifications or naturally occurring modifications such as phosphorylation, glycosylation, myristylation and the like.

The polypeptide may be provided in any form and at any degree of purification, from tissues, fluids or cells comprising said polypeptide in an endogenous form, more preferably cells overexpressing the polypeptide, crude or enriched lysates of such cells, to purified and/or isolated polypeptide which may be essentially pure.

The term "SARS-CoV-2 spike protein", refers to the S protein of the Wuhan strain (MN908947.3, from 18 March 2020) comprising the sequence identified here as SEQ ID NO: 77. It may also refer to the S protein of any of the following lineages and variants
- Lineage B.1.1.7 / Variant of Concern Alpha (20-DEC-01) First detected in October 2020 during the COVID-19 pandemic in the United Kingdom Lineage B.1.1.7, was previously known as the first Variant Under Investigation in December 2020 (VUI - 202012/01) and later notated as VOC-202012/01. It is also known as lineage B.1.1.7 or 201/501 Y.V1 (formerly 2p.337 0B/501Y.V1). As of May 2021, Lineage B.1 .1.7 has been detected in some 120 countries.
- Variant of Concern 21 FEB-02: Previously written as VOC-202102/02, described by Public Health England (PHE) as "B.1.1.7 with E484K" is of the same lineage in the Pango nomenclature system, but has an additional E484K mutation.
- Lineage B.1.1.207: First sequenced in August 2020 in Nigeria, this variant has a P681 H mutation, shared in common with UK's Lineage B.1 .1 .7. It shares no other mutations with Lineage B.1.1.7 and as of late December 2020 this variant accounts for around 1% of viral genomes sequenced in Nigeria. As of May 2021, Lineage B.1.1.207 has been detected in 10 countries.
- Lineage B.1.1.317: B.1.1.317 was identified in Queensland, Australia.
- Lineage B.1 .1.318 was designated by PHE as a VUI (VUI-21 FEB-04, previously VU 1-202102/04) on 24 February 2021.
- Lineage B.1.351 (Beta) On 18 December 2020, the 501. V2 variant, also known as 501. V2, 20H/501Y.V2 (formerly 20C/501Y.V2), VOC-20DEC-02 (formerly VOC-202012/02), or lineage B.1.351, was first detected in South Africa and reported by the country's health department. The variant contains several mutations that allow it to attach more easily to human cells because of the following three mutations in the receptor-binding domain (RBD) in the spike glycoprotein of the virus: N501Y, K417N, and E484K.
- Lineage B.1.429 / CAL.20C (Epsilon) (comprising the sequence identified here as SEQ ID NO: 21).
- Lineage B.1.429, also known as CAL.20C, is defined by five distinct mutations (I4205V and D1183Y in the ORF1ab-gene, and S13I, W152C, L452R in the spike protein's S-gene). B.1.429 was first observed in July 2020 by researchers at the Cedars-Sinai Medical Center, California, in one of 1 ,230 virus samples collected in Los Angeles County since the start of the COVID-19 epidemic.
- Lineage B.1.525 (Eta), also called VUI-21 FEB-03[15] (previously VU 1-202102/03) by Public Health England (PHE) and formerly known as UK1188, does not carry the same N501Y mutation found in B.1.1.7, 501. V2 and P.1, but carries the same E484K-mutation as found in the P.1 , P.2, and 501. V2 variants, and also carries the same AH69/AV70 deletion (a deletion of the amino acids histidine and valine in positions 69 and 70) as found in B.1 .1 .7, N439K variant (B.1.141 and B.1.258) and Y453F variant (Cluster 5). B.1.525 differs from other variants by having both the E484K-mutation and a new F888L mutation (a substitution of phenylalanine (F) with leucine (L) in the S2 domain of the spike protein).
- Lineage B.1.526 (Iota): In November 2020, a mutant variant was discovered in New York City, which was named B.1.526.
- Lineage B.1.617.1 (Kappa): In October 2020, a new variant was discovered in India, which was named B.1.617.1. Among some 15 defining mutations, it has spike mutations D111 D (synonymous substitution), G142D, P681 R, E484Q and L452R, the latter two of which may cause it to easily avoid antibodies. Public Health England (PHE) designated B.1.617 as a 'Variant under investigation', VUI-21 APR-01. On 29 April 2021, PHE added two further variants, VUI-21 APR- 02 and VUI-21APR-03, effectively B.1.617.2 and B.1.617.3. B.1.617.2 (which notably lacks mutation at E484Q; Variant Delta), (comprising the sequence identified here as SEQ ID NO: 23) is a "variant of concern".
- Lineage B.1.618: This variant was first isolated in October 2020, and has the E484K mutation as in South African variant B.1.351.
- Lineage P.1 : Lineage P.1, termed Variant of Concern 21 JAN-02 (formerly VOC-202101/02) (Gamma) by Public Health England and 20J/501Y.V3 by Nextstrain, was detected in Tokyo on 6 January 2021 by the National Institute of Infectious Diseases (NIID). This variant of SARS-CoV-2 has been named in the P.1 lineage, and has 17 unique amino acid changes, 10 of which in its spike protein, including N501Y, E484K and K417T.
- Lineage P.3 (Teta): On 18 February 2021, the Department of Health of the Philippines confirmed the detection of two mutations of COVID-19 in Central Visayas after samples from patients were sent to undergo genome sequencing. The mutations were later named as E484K and N501Y.
- Lineage B.1.1.529 (Omicron), Sudafrica, GISAID clade GRA.
- Lineage C.37 (Lambda) Peru, GISAID clade GR/452Q.V1.
- Lineage B.1.621 (Mu), Colombia, GISAID clade GH.

The term "region" when referred to the SARS-CoV-2 spike protein is used to indicate that the polypeptides of the invention do not contain the complete SARS-CoV-2 spike protein but a fragment thereof. The length of the region is not particularly limitative of the scope of the invention. In some embodiments, the polypeptide of the invention comprises a sequence derived from the SARS-CoV-2 spike protein which is 1200 amino acids or less, 1150 amino acids or less, 1100 amino acids or less, 1000 amino acids or less or 950 amino acids or less. In some embodiments, the polypeptide of the invention comprises, consists or essentially consist of amino acids 14-1213 of the SARS-CoV-2 spike protein precursor protein, which corresponds to the ectodomain of the spike protein relative to the spike precursor protein shown in the UniProt database under accession number P0DTC2 (release of 01 March 2023). In some embodiments, the polypeptide of the invention comprises a region of the SARS-CoV-2 spike protein which starts at position 161, at position 150, at position 140, at position 130, at position 120, at position 110, at position 90, at position 80, at position 60, at position 50, at position 40, at position 30, at position 20 or at position 14 of said SARS-CoV-2 spike protein.

In some embodiments, the polypeptide of the invention comprises a region of the SARS-CoV-2 spike protein which ends at position 1050, at position 1100, at position 1110, at position 1120, at position 1130, at position 1140, at position 1150, at position 1160, at position 1170, at position 1180, at position 1190, at position 1200 or at position 1210 of said SARS-CoV-2 spike protein.

In certain embodiments, the proteins according to the invention are soluble proteins, e.g. S protein ectodomains, and comprise a truncated S2 domain. As used herein a "truncated" S2 domain refers to a S2 domain that is not a full length S2 domain, i.e. wherein either N-terminally or C-terminally one or more amino acid residues have been deleted. 1). Preferably, the region of the SARS-CoV-2 spike protein forming part of the polypeptides according to the present invention lacks at least the C-terminal tail (CT) of the SARS-CoV-2 spike protein or lacks both the transmembrane region (TM) and/or a C-terminal tail (CT) of the SARS-CoV-2 spike protein. In some embodiments, the region of the SARS-CoV-2 spike protein forming part of the polypeptides according to the present invention consist or essentially consist of the SARS-CoV-2 spike protein ectodomain.

The term "transmembrane region (TM)" of the SARS-CoV-2 spike protein as defined herein is a hydrophobic domain that is inserted into the cell membrane such that the parts of the protein on either side of the region are on opposite sides of the membrane. It comprises positions 1214 - 1234 of the Spike protein as defined in SEQ ID NO: 77

The term "C-terminally" or "C-terminal" or "carboxyl-terminal" or "C-terminal tail" of the SARS-CoV-2 spike protein are used herein to denote positions within polypeptides. Where the context allows, these terms are used with reference to a particular sequence or portion of a polypeptide to denote proximity or relative position. For example, a certain sequence positioned carboxyl- terminal to a reference sequence within a polypeptide is located proximal to the carboxyl terminus of the reference sequence, but is not necessarily at the carboxyl terminus of the complete polypeptide. It comprises positions 1235 to 1273 of the Spike protein as defined in SEQ ID NO: 77.

The term "ectodomain of the SARS-CoV-2 spike protein as defined herein is the region of the spike protein which extends into the extracellular space. It extends from position 13 to 1213 of the Spike protein as defined in SEQ ID NO: 77. In some embodiments, the region of the SARS-CoV-2 spike protein forming part of the polypeptides according to the present invention contains the complete extracellular domain of the SARS-CoV-2 spike protein. In other embodiments, said region contains a region of the extracellular domain formed by 1200 amino acids or less, 1150 amino acids or less, 1100 amino acids or less, 1000 amino acids or less, 950 amino acids or less.

Accordingly, in one embodiment, the polypeptides of the present invention derive from the extracellular region of the SARS-CoV-2 spike protein and do not contain other regions of the protein which are found in the native SARS-CoV-2 spike protein.

The variants according to the present invention are defined by the different mutations with respect to the native protein, wherein the position of each mutation is defined by its position taking as reference the polypeptide of SEQ ID NO:77 and which corresponds to the sequence shown in the UniProt database under accession number P0DTC2 (release of 01 March 2023).

As used herein, "mutation" refers to a change in the sequence of a polypeptide molecule as compared to a reference or wild-type polypeptide molecule, respectively. A mutation can result in several different types of change in sequence, including exclusively amino acid(s) substitution.

A "functionally equivalent variant of the polypeptides of the invention can be an amino acid sequence derived from any of the polypeptides of the invention comprising the addition, substitution or modification of one or more amino acid residues. By way of illustration, equivalent variants of the sequences any of the polypeptides of the invention include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the polypeptide according to the invention and/or comprising the addition of 1 amino acid, 2 amino acids, 3 amino acids, 4 amino acids, 5 amino acids, 10 amino acids, 11 amino acids, 12 amino acids, 13 amino acids, 14 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxy terminus of the polypeptide according to the invention, and maintaining at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, or at least 100% of the activity of the sequence of the polypeptide according to the invention.

The activity or function of the functionally equivalent variant of the polypeptide according to the invention can also be determined by assaying the immunogenic activity of the polypeptides by a method shown in the examples of the present application.

"Functionally equivalent variants" of the polypeptide according to the invention also include amino acid sequences with a sequence identity of at least 70%, at least 75%, at least 80%, at least 85%, at least 90%, at least 91 %, at least 92%, at least 93%, at least 94%, at least 95%, at least 96%, at least 97%, at least 98%, or at least 99% with the sequence of any of the polypeptides as defined herein.

The terms "identity", "identical" or "percent identity" in the context of two or more amino acids or nucleotide sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first amino acid sequence to a second amino acid sequence is calculated as 100 × (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

As a non-limiting example, whether any particular polypeptide has a certain percentage sequence identity (e.g., is at least 80% identical, at least 85% identical, at least 90% identical, and in some embodiments, at least 95%, 96%, 97%, 98%, or 99% identical) to a reference sequence. For instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

The term "immunologically active variant" refers to a polypeptide that comprises at least one antigen, which elicits an immunological response in the host to which the immunogenic polypeptide is administered. Such immunological response may be a cellular and/or antibody-mediated immune response to the immunogenic polypeptide of the invention. Preferably, the immunogenic polypeptide induces an immune response and, more preferably, confers protective immunity against one or more of the clinical signs of a SARS-CoV-2 infection. The host is also described as "subject". Preferably, any of the hosts or subjects described or mentioned herein without limitation human or mammal. Usually, an "immunological response" includes but is not limited to one or more of the following effects: the production or activation of antibodies, B cells, helper T cells, suppressor T cells, and/or cytotoxic T cells and/or gamma-delta T cells, directed specifically to an antigen or antigens included in the immunogenic composition of the invention. Preferably, the host will display either a protective immunological response or a therapeutically response.

The term "immunologically active variant" is understood to mean all those immunologically active polypeptides derived from the sequences of the polypeptides of the invention by modification, substitution, insertion and/or deletion of one or more amino acids, whenever the function of eliciting an immunological response in the host to which the immunologically active polypeptide is administered is substantially maintained. The term "immunologically active variant" as used herein also refers to a variant of a polypeptide that retains substantially equivalent ability to induce an immune response in a subject as the reference polypeptide. The term "immunologically active variant" is well understood in the art and is further defined in detail herein. Immunologically functional equivalents may increase the antigenicity of a polypeptide, maintain the same level of antigenicity of the reference polypeptide, or decrease the antigenicity of a polypeptide only slightly so that it maintains its usefulness as an antigen in an immunogenic composition.

The term "immunologically active variant", as used herein, refers to variants which are able to generate an immune response which differs from the immune response generated with the native region by no more than 5 %, no more than 10 %, no more than 15 %, no more than 20 %, no more than 25 %, no more than 30 %, no more than 35 %, no more than least 40 %, no more than 45 %, no more than 50%, no more than 55 %, no more than 60 %, no more than 65 %, no more than 70 %, no more than 75 %, no more than 80 %, no more than 85 %, no more than 90 % or no more than 95 %. For the purposes of the present invention, a polypeptide that is useful as an antigen in an immunogenic composition (i.e. has sufficient "immunogenic activity") can be identified by any method commonly known in the art for measuring the ability of a given polypeptide to trigger the generation of antibodies specific for said polypeptide when administered to a host organism. The ability of a given variant of the polypeptide of the invention to be an immunologically equivalent variant may be determined using standard neutralization assays, wherein the suspected variant is inoculated into a test animal and the resulting antibodies are tested for their ability to neutralize the infection of susceptible cells by SAR-CoV-2 virus. Any known and/or conventional method for screening and identifying and obtaining the neutralizing antibodies of the invention are contemplated by the present invention. In certain examples, a cell line or pseudovirus assay is used as a neutralization assay. Such assays are well known in the art.

Accordingly, "immunologically functional equivalent" of a polypeptide according to the invention shows a degree of identity with respect to the amino acid sequence of the polypeptide according to the invention of at least 50%, at least 55%, at least 60%, at least 65%, at least 70%, at least 75%, at least 80%, at least 85%, at least 90%, at least 95%, at least 96%, at least 97%, at least 98% or at least 99% provided the immunogenic activity of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art.

As an example of modifications contemplated to be within the scope of the present invention, certain amino acids may be substituted for other amino acids in a polypeptide structure without appreciable loss of interactive binding capacity of the structure such as, for example, the epitope of an antigen that is recognized and bound by an antibody. Since it is the interactive capacity and nature of a polypeptide that defines its biological (e.g. immunological) functional activity, certain amino acid sequence substitutions can be made in an amino acid sequence (or its underlying DNA coding sequence) and nevertheless obtain a polypeptide with comparable properties. Various changes may be made to the amino acid sequences of the antigens of the present invention without appreciable loss of immunogenic activity.

It is understood in the art that in order to make functionally equivalent amino acid substitutions, the hydropathic index of amino acids may be considered. The importance of the hydropathic amino acid index in conferring interactive biological function on a polypeptide is generally understood in the art. It is known that certain amino acids may be substituted for other amino acids having a similar hydropathic index or score and still retain a similar biological activity. In making changes based upon the hydropathic index, the substitution of amino acids whose hydropathic indices are within plus or minus 2 is preferred, those which are within plus or minus 1 are particularly preferred, and those within plus or minus 0.5 are even more particularly preferred. Each amino acid has been assigned a hydropathic index on the basis of its hydrophobicity and charge characteristics; these are: isoleucine (+4.5), valine (+4.2), leucine (+3.8), phenylalanine (+2.8), cysteine/cystine (+2.5), methionine (+1.9), alanine (+1.8), glycine (-0.4), 5 threonine (-0.7), serine (-0.8), tryptophan (-0.9), tyrosine (-1.3), proline (-1.6), histidine (-3.2), glutamate (-3.5), glutamine (-3.5), aspartate (-3.5), asparagine (-3.5), lysine (-3.9) and arginine (-4.5). It also is understood in the art that the substitution of similar amino acids can be made effectively on the basis of hydrophilicity; particularly where the immunologically functional equivalent polypeptide thereby created is intended for use in immunological embodiments, as in certain embodiments of the present invention. The greatest local average hydrophilicity of a protein, as governed by the hydrophilicity of its adjacent amino acids, correlates with its immunogenicity and antigenicity. In making changes based upon similar hydrophilicity values, the substitution of amino acids whose hydrophilicity values are within plus or minus 2 is preferred, those which are within plus or minus 1 are particularly preferred, and those within plus or minus 0.5 are even more particularly preferred. The following hydrophilicity values have been assigned to amino acid residues: arginine (+3.0), lysine (+3.0), aspartate (+3.0 plus or minus 1), glutamate (+3.0 plus or minus 1), serine (+0.3), asparagine 20 (+0.2), glutamine (+0.2), glycine (0), threonine (-0.4), proline (-0.5 plus or minus 1), alanine (-0.5), histidine (-0.5), cysteine (-1.0), methionine (-1.3), valine (-1.5), leucine (-1.8), isoleucine (-1.8), tyrosine (-2.3), phenylalanine (-2.5) and tryptophan (-3.4).

In particular embodiments, the polypeptide of the invention comprises a plurality of mutations, wherein the different variants are characterized by the presence of one of the following sets of mutations (positions relative to the sequence of SEQ ID NO:77):
- A372W and D420R (the S-2M mutant);
- D198F, G232L, A372W, N394Q and D420R (the S-5M mutant);
- Q755K, L849K, A892N, K947R and K1045R (the S-22 mutant);
- S758E, T912R and K947R (the S-29 mutant);
- A706Q, S758E, L849K, V963E, S1030R and Q1113R (the S-21 mutant);
- S758E, L849K and V963E (the S-24 mutant);
- Q755K, C851Q and T961E (the S-26 mutant);
- A706Q, S1030R and Q1113R (the S-27 mutant); and
- S758E, T912R and K947R (the S-29 mutant).

In a particular embodiment, the polypeptide of the invention further comprises the K986P and/or the V987P mutations relative to the SARS-CoV-2 spike protein sequence as defined in SEQ ID NO: 77 which have been shown to improve the stability of the prefusion conformation.

In one embodiment, the polypeptide of the invention comprises the V987H mutation and a K986P mutation relative to the SARS-CoV-2 spike protein sequence as defined in SEQ ID NO: 77.

In another embodiment, the polypeptide of the invention comprises the L849K mutation and the K986P and V987P mutations relative to the SARS-CoV-2 spike protein sequence as defined in SEQ ID NO: 77. In yet another embodiment, the polypeptide of the invention comprises the S758E mutation and the K986P and V987P mutations relative to the SARS-CoV-2 spike protein sequence as defined in SEQ ID NO: 77.

In another preferred embodiment, the polypeptide according to the invention comprises a sequence as defined in any of SEQ ID NO: 1 to 21.

In certain embodiments, the polypeptides according to the present invention have been modified in order to delete the endogenous furin cleavage site. As used herein the term "furin cleavage site" refers to any sequence having the X(K/R)R (RKR or RRR) consensus motif. The site is present in the SARS-CoV-2 spike protein and is responsible for the proteolytic cleavage of the spike protein at the monobasic S1/S2 cleavage site, RRARS (SEQ ID NO: 25). This cleavage is essential for spike protein-mediated cell-cell fusion and entry into human lung cells.

Deleting the furin cleavage site may be achieved in any way known in the art. In certain embodiments, the deletion of the furin cleavage site comprises introducing a mutation of the amino acid at position 682 into S and/or a mutation of the amino acid at position 685 into G of the Spike protein as defined in SEQ ID NO: 77. In a more preferred embodiment, the naturally occurring furin cleavage site has been mutated from the naturally occurring RRARS sequence (SEQ ID NO:80) at positions 682 to 686 to the to the GSASS sequence, as defined in SEQ ID NO: 72.

In a particular embodiment, the polypeptide further comprises a trimerization motif located C-terminally within the polypeptide of the invention.

The term "trimerization motif" is understood as an amino acid sequence whose function is to promote folding and trimerization of monomer polypeptides. Examples of suitable trimerization domains include without restriction, the T4 bacteriophage foldon trimerization domain, the trimerization domain of collagen XV or the trimerization domain of collagen XVIII. In a preferred embodiment, the trimerization domain is the T4 bacteriophage foldon as defined in SEQ ID NO: 22.

The trimerization domain may be directly connected to the C-terminus of the polypeptide according to the invention or may be connected through a linker region.

The term "linker" or "spacer", as used herein, refers to a peptide, oligopeptide or polypeptide which is used to connect to regions within a polypeptide. There is no standard definition regarding the size boundaries between what is meant by peptide, oligopeptide and polypeptide, but typically a peptide may be viewed as comprising between 2-20 amino acids, and oligopeptide between 21-39 amino acids and a polypeptide may be viewed as comprising at least 40 amino acids. Thus, a peptide linker as defined herein may be viewed as comprising at least 6 amino acids, e.g. 6-300 amino acids. In this sense, a preferred spacer would be a hinge region characterized by a structural ductility or flexibility allowing this movement. The length of the spacer can vary; typically, the number of amino acids in the spacer is 100 or less amino acids, preferably 50 or less amino acids, more preferably 40 or less amino acids, still more preferably, 30 or less amino acids, or even more preferably 20 or less amino acids. Additional linkers that can be used in the instant invention include the peptides of the amino acid sequences GAP, AAA. In a particular embodiment, said spacer is a peptide having structural flexibility (i.e., a flexible linking peptide or "flexible linker") and comprises 2 or more amino acids selected from the group consisting of glycine, serine, alanine and threonine. In another particular embodiment, the spacer is a peptide containing repeats of amino acid residues, particularly Gly and Ser, or any other suitable repeats of amino acid residues. Virtually any flexible linker can be used as spacer according to this invention. In a preferred embodiment, the spacer is a flexible linker. In a more preferred embodiment, the flexible linker is between 1 and 18 residues. In a still more preferred embodiment, the flexible linker is 5, 17 or 18 residues, preferably 18 residues.

Preferred examples of linker peptides comprise 2 or more amino acids selected from the group consisting of glycine, serine, alanine and threonine. A preferred example of a flexible linker is a polyglycine linker. The possible examples of linker/spacer sequences include GGSSGGS (SEQ ID NO: 81), SGGTSGSTSGTGST (SEQ ID NO: 82), AGSSTGSSTGPGSTT (SEQ ID NO: 83) or GGSGGAP (SEQ ID NO: 84). These sequences have been used for binding designed coiled coils to other protein domains [Muller, K.M., Arndt, K.M. and Alber, T., Meth. Enzymology, 2000, 328: 261-281]. Further non-limiting examples of suitable linkers comprise the amino acid sequence GGGVEGGG (SEQ ID NO: 85), the sequence of 10 amino acid residues of the upper hinge region of murine IgG3 (PKPSTPPGSS, SEQ ID NO: 86), which has been used for the production of dimerized antibodies by means of a coiled coil (Pack, P. and Pluckthun, A., 1992, Biochemistry 31:1579-1584), the peptide of sequence APAETKAEPMT (SEQ ID NO: 87), the peptide of sequence GAP, the peptide of sequence AAA and the peptide of sequence AAALE (SEQ ID NO: 88). In a preferred embodiment, the linker connecting the region of the spike protein and the trimerization domain is the sequence defined in SEQ ID NO: 79.

Alternatively, a suitable spacer can be based on the sequence of 10 amino acid residues of the upper hinge region of murine IgG3; which has been used for the production of dimerized antibodies by means of a coiled coil (Pack P. and Pluckthun, A., 1992, Biochemistry 31:1579-1584) and can be useful as a spacer peptide according to the present invention. It can also be a corresponding sequence of the upper hinge region of human IgG3 or other human Ig subclasses (lgG1, IgG2, IgG4, IgM and IgA). The sequences of human Igs are not expected to be immunogenic in human beings.

In another preferred embodiment, the polypeptide according to the invention comprises a sequence as defined in any of SEQ ID NO: 103 to 123.

In a particular embodiment, the polypeptide of the invention further comprises one or more tags, known as "affinity tag", which can be used for the detection or for the purification of the polypeptides of the invention by using a ligand which is capable of specifically binding to the affinity tag. In one embodiment, the polypeptides of the invention comprise a first affinity tag which can be used for the purification of the polypeptide and a second affinity tag which is used for detection of the polypeptide.

As used herein, the term "affinity tag" refers to an amino acid sequence, which has affinity for a specific reagent and which can be used either to separate the polypeptides from a pool of polypeptides or for the specific detection of the polypeptide. The "affinity tag" allows isolation from other components on the basis of its affinity for the binding partner. The affinity tag may form part of a target polypeptide sequence to be purified. Where the affinity tag forms part of the target polypeptide, the tag may remain part of the target polypeptide following purification and not be cleaved therefrom.

Examples of affinity domains include His5 (HHHHH) (SEQ ID NO: 96), His X6 (HHHHHH) (SEQ ID NO: 23), C-myc (EQKLISEEDL) (SEQ ID NO: 97), Flag (DYKDDDDK) (SEQ ID NO: 98), StrepTag (WSHPQFEK) (SEQ ID NO: 24), hemagglutinin, e.g., HA Tag (YPYDVPDYA; SEQ ID NO: 99), glutathinone-S-transferase (GST), thioredoxin, cellulose binding domain, RYIRS (SEQ ID NO: 100), Phe-His-His-Thr (SEQ ID NO: 101), chitin binding domain, S-peptide, T7 peptide, SH2 domain, C-end RNA tag, WEAAAREACCRECCARA (SEQ ID NO: 102), the Twin-Strep-tag (WSHPQFEKGGGSGGGGSGGSAWSHPQFEK) (SEQ ID NO:89), metal binding domains, e.g., zinc binding domains or calcium binding domains such as those from calcium-binding proteins, e.g., calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D9K, calbindin D28K, and calretinin, inteins, biotin, streptavidin, MyoD, leucine zipper sequences, and maltose binding protein.

The affinity tag may be directly linked to the polypeptide or connected via an amino acid linker. Suitable linkers that can be used to connect the affinity tag with the other part of the polypeptide are as defined above in the context of linkers suitable for contacting the region of the SARS-CoV-2 protein and the trimerization domain and are equally applicable for the purposes of contacting the affinity tag and the rest of the polypeptide.

Preferably, the affinity tag comprises a histidine rich region. Preferably, the affinity tag comprises at least 4, 5 or 6 histidine residues. More preferably, the polypeptide of the invention comprises a hexahistidine region as defined in SEQ ID NO: 23 and a Streptag II as defined in SEQ ID NO: 24. In some embodiments, the polypeptide of the invention comprises a two Streptag II regions separated by a linker region. In some embodiments, the two Streptag II regions separated by a linker region has a sequence as defined in SEQ ID NO: 89. In some embodiments, the polypeptide of the invention comprises a first affinity tag which is preferably an hexahistidine tag and a second affinity tag which two Streptag II regions separated by a linker region has a sequences as defined in SEQ ID NO: 89. In a more preferred embodiment, the first tag and the second tag are connected by a SA dipeptide.

In another embodiment, the polypeptide of the invention comprises a cleavage site located between the trimerization domain and the affinity or affinity tags which allows the detachment of the affinity tag from the target protein. Selective cleavage sites and methods of cleaving such sequences are well known in the art. The affinity tag may be attached to the target peptide at the C-terminal or the N-terminal end thereof. The cleavage site is usually a target site for a protease.

The term "target site", as used herein, refers to a polypeptide sequence which can be recognized and processed by an enzyme when the target site and the enzyme are contacted under suitable conditions. In this case, the enzyme is a protease. Suitable protease target sites for use in the present invention and the corresponding protease include:
- The LVPRGS target site as defined in SEQ ID NO: 71, which is cleaved by thrombin,
- The ENLYFQG/S target site as defined in SEQ ID NO: 72 or SEQ ID NO: 73 which is cleaved by the TEV protein from Tobacco Etch Virus,
- The IE/DGR target site as defined in SEQ ID NO: 74 or SEQ ID NO: 75 which is processed by factor Xa,
- The LEVLFQGP target site as defined in SEQ ID NO: 76 which is processed by the PreScission Protease (rPSP) from human rhinovirus (HRV 3C))
- The RRARS target site as defined in SEQ ID NO: 25 which is processed by furin and
- The LEVLFQGP target site having the sequence of SEQ ID NO: 26 which is processed by the HRV C3 protease.

In a particular embodiment, the polypeptide according to the invention comprises a sequence selected from the group consisting of SEQ ID NO: 27 to 47.

In another embodiment, the invention relates to a trimeric polypeptide that results from the assembly of three copies of the polypeptide of the invention wherein each of the monomer polypeptides comprises a trimerization motif.

The term "trimeric polypeptide" or "trimer" refers to a structure comprising three monomers, for example, three monomer polypeptides. The term "homotrimer" refers to a structure comprising three identical monomers, for example, three identical monomer polypeptides according to the invention. The ability to trimerize can be assessed experimentally, for example, by SDS-PAGE in non-reducing conditions followed by Coomassie-blue staining, by which the relative abundance and molecular masses of trimeric forms with respect to monomeric forms can be visualized and compared.

### Polynucleotides, vectors and host cells:

In a second aspect the present invention relates to a polynucleotide encoding the polypeptide of the first aspect.

As used herein, the term "polynucleotide" refers to a polymer composed of a multiplicity of nucleotide units (deoxyribonucleotides or ribonucleotides, or related structural variants or synthetic analogues thereof) linked via phosphodiester bonds (or related structural variants on synthetic analogues thereof). The term polynucleotide includes double or single stranded genomic and cDNA, RNA, any synthetic and genetically manipulated polynucleotide, and both sense and anti-sense polynucleotide (although only sense stands are being disclosed in the present invention). This includes single- and doublestranded molecules, i.e., DNA-DNA, DNA-RNA and RNA-RNA hybrids.

In a preferred embodiment, the polynucleotide further comprises a sequence encoding a signal peptide in the same open reading frame as the polypeptide. More preferably the sequence encoding a signal peptide is selected from SEQ ID NO:48 or SEQ ID NO:49.

The term "signal peptide", as used herein, refers to a protein sequence comprising, or consisting essentially of, or yet further consisting of MDWTWRFLFVVAAATGVQS (SEQ ID NO:48), or of MDWTWRFLFVVAAATGVQS (SEQ ID NO:49).

In another preferred embodiment, the polynucleotide comprises a sequence optimized for expression in mammalian cells. Preferably, the polynucleotide comprises the sequences according to SEQ ID NO: 50 to 71. The optimization of the sequence for expression in mammalian cells may be done using standard techniques known by the person skilled in the art and are also described in the state of the art.

In a third aspect, the present invention relates to a vector comprising the polynucleotide of the second aspect of the invention.

The term "vector" denotes a nucleic acid molecule into which a second nucleic acid molecule can be inserted for introduction into a host where it will be replicated, and in some cases expressed. In other words, a vector is capable of transporting a nucleic acid molecule to which it has been linked. Cloning as well as expression vectors are contemplated by the term "vector", as used herein. Vectors include, but are not limited to, plasmids, cosmids, bacterial artificial chromosomes (BAC) and yeast artificial chromosomes (YAC) and vectors derived from bacteriophages or plant or animal (including human) viruses. Vectors comprise an origin of replication recognised by the proposed host and in case of expression vectors, promoter and other regulatory regions recognised by the host. A vector containing a second nucleic acid molecule is introduced into a cell by transformation, transfection, or by making use of viral entry mechanisms. Certain vectors are capable of autonomous replication in a host into which they are introduced (e.g., vectors having a bacterial origin of replication can replicate in bacteria). Other vectors can be integrated into the genome of a host upon introduction into the host, and thereby are replicated along with the host genome.

In a fourth aspect the present invention relates to a host cell comprising the vector according to the third aspect of the invention.

Non-limiting examples of vectors that can be used to express the polypeptides according to the invention include any of the vectors explained above in the context of the vectors according to the invention such as viral-based vectors (e.g., retrovirus, adenovirus, adeno-associated virus, lentivirus), baculovirus vectors (see, Examples), plasmid vectors, non-viral vectors, mammalians vectors, mammalian artificial chromosomes (e.g., liposomes, particulate carriers, etc.) and combinations thereof.

In a preferred embodiment, the expression system is a mammalian cell line culture.

The term "host cell", as used herein, refers to a cell into which a nucleic acid of the invention, such as a polynucleotide or a vector according to the invention, has been introduced and is capable of expressing the polypeptides of the invention as monomers or trimers or a combination thereof. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal. For example, reporter constructs, as described above, can provide a selectable or screenable trait upon activation or inhibition of gene transcription in response to a transcriptional regulatory protein; in order to achieve optimal selection or screening, the host cell phenotype will be considered. A host cell of the present invention includes prokaryotic cells and eukaryotic cells. Prokaryotes include gram-negative or gram-positive bacteria, for example, E. coli or Bacilli. It is to be understood that prokaryotic cells will be used, preferably, for the propagation of the transcription control sequence comprising polynucleotides or the vector of the present invention. Suitable prokaryotic host cells for transformation include, for example, E. coli, Bacillus subtilis, Salmonella typhimurium, and various other species within the genera Pseudomonas, Streptomyces, and Staphylococcus. Eukaryotic cells include, but are not limited to, yeast cells, plant cells, fungal cells, insect cells (e.g., baculovirus), mammalian cells, and the cells of parasitic organisms, e.g., trypanosomes. As used herein, yeast includes not only yeast in a strict taxonomic sense, i.e., unicellular organisms, but also yeast-like multicellular fungi of filamentous fungi. Exemplary species include Kluyverei lactis, Schizosaccharomyces pombe, and Ustilaqo maydis, with Saccharomyces cerevisiae being preferred. Other yeasts which can be used in practicing the present invention are Neurospora crassa, Aspergillus niger, Aspergillus nidulans, Pichia pastoris, *Candida tropicalis,* and *Hansenula polymorpha.* Mammalian host cell culture systems include primary cells as well as established cell lines such as COS cells, L cells, 3T3 cells, Chinese hamster ovary (CHO) cells, embryonic stem cells, with BHK, HeK or HeLa, insect cells, for example but not restricted to, MDCK, BHK, VERO, MRC-5, WI-38, HT1080, 293, 293T, RD, COS-7, CHO, Jurkat, HUT, SUPT, C8166, MOLT4/clone8, MT-2, MT-4, H9, PM1, CEM, myeloma cells (e.g., SB20 cells) and CEMX174 (such cell lines are available, for example, from the ATCC). Similarly, bacterial hosts such as E. coli, Bacillus subtilis, and Streptococcus spp., will find use with the present expression constructs. Yeast hosts useful in the present disclosure include inter alia, *Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Fungal hosts include, for example, Aspergillus.

Insect cells for use with baculovirus expression vectors include, inter alia, *Aedes aegypti, Autographa californica, Bombyx mori, Drosophila melanogaster, Spodoptera frugiperda,* and *Trichoplusia ni.*

### Method for the production of polypeptide and for the production of anti-SASR-CoV2 antibodies:

The authors have also developed methods for expressing the polypeptides of the invention using expression systems and, when the polypeptides contain a trimerization motif, for obtaining trimers containing the polypeptides of the invention. In particular, the expression is carried out using suitable expression vectors which, when inserted into a mammalian cell line, allow the cell line to express the polypeptides according the invention. When the polypeptides are expressed from a polynucleotide which encodes the polypeptide according to the invention fused in frame to a signal sequence and containing a trimerization domain, the polypeptides are secreted into the cell culture media in their trimeric form, from which they can be purified. If the polypeptide according to the invention contains a protease target site, the trimers can also be treated with the protease in order to remove the affinity tag or tags as explained above.

Thus, in a further aspect, the invention relates to a method for producing a polypeptide according to the first aspect of the invention or a trimer according to the invention, which comprises the steps of:
(i) expressing in a suitable expression system a polynucleotide according to the second aspect of the invention and;
(ii) recovering the polypeptides from the expression system.

The method for producing a polypeptide according to the invention or a trimer according to the invention comprises a first step in which the polypeptides are expressed in a suitable expression system. The expression is preferably achieved by introducing the polynucleotide according to the invention in a suitable expression system.

In a preferred embodiment, if the polypeptide comprises a trimerization motif, then the expression is carried out under conditions adequate for allowing the trimerization of said polypeptides, so that trimer polypeptides are formed in step (i).

In a second step, the method for producing a polypeptide or a trimer according to the invention involves recovering the polypeptides from the expression system.

The recovery can be carried out using any method known in the art. In particular, the recovery can be carried out using any suitable method of protein purification, for example and without limitation, chromatography, in particular, affinity chromatography.

In a one embodiment, if the polynucleotide comprises a sequence encoding a signal peptide, then the polypeptide is recovered from the supernatant of the expression system.

The term "supernatant", as used herein, refers to a medium that has been used to support growth of cultured cells. Generally, herein, the cells have been infected with SARS-CoV-2 and the cell culture supernatant contains active progeny SARS-CoV-2 produced by the infected cells.

In some embodiments, if the polypeptides comprise an affinity tag, then the recovery step (ii) is carried out by contacting the expression system or the supernatant thereof, as the case may be, with a ligand with the capacity to bind to the affinity tag and recovering those polypeptides or trimers which are bound to the ligand.

In a more preferred embodiment, in those cases in which the polypeptides of the invention contain a cleavage site between the trimerization domain and the affinity tags, then the expressed polypeptide or trimeric polypeptides are contacted with a protease which is capable of recognizing and cleaving the target site found in the monomers, thereby producing trimeric polypeptides devoid of the affinity tags.

### Therapeutic methods

As it is shown in the examples of the present application, the authors of the invention have found out that the trimeric polypeptides described in the invention are able to elicit an immune response in a subject, being therefore useful tools for preventing or treating subjects against a disease caused by the infection by SARS-CoV-2. The examples demonstrate that immunization with the polypeptide of the invention induces anti-RBD antibodies with a neutralizing capacity against SARS-CoV-2 comparable to a standard purified neutralizing monoclonal antibody against SARS-CoV-2.

Accordingly, in another aspect, the invention relates to a polypeptide according to the invention, a polynucleotide according to the invention, a trimeric polypeptide according to the invention or a vector according to the invention for use in the treatment or prevention of a disease caused by the infection by SARS-CoV-2.

The terms "prevent", "preventing", and "prevention", as used herein, refer to a decrease in the occurrence of pathological cells in an animal. The prevention may be complete (e.g. the total absence of pathological cells in a subject). The prevention may also be partial, such that for example the occurrence of pathological cells in a subject is less than that which would have occurred without the present invention. Prevention also refers to reduced susceptibility to a clinical condition.

The term "treatment", as used herein, comprises any type of therapy, which aims at terminating, preventing, ameliorating and/or reducing the susceptibility to a clinical condition as described herein, e.g. SARS-Co-V-2 infection and related conditions. Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "SARS-Co-V-2 infection", as used herein, refers not only to symptoms of the respiratory pathways (i.e. from shortness of breath to pneumonia) but also to a broad group of clinical symptoms affecting a variety of tissues and organs upon infection by SARS-CoV-2, such as organ failure, heart problems, acute respiratory distress syndrome, blood clots, acute kidney injury and additional opportunistic viral and bacterial infections.

### Immunogenic composition and vaccine of the invention:

The authors of the present invention have shown that the different variant polypeptides are capable of eliciting an immune response when administered to the test animals, thereby inducing a remarkably strong humoral immune response against the Spike protein, including the RBD. In addition, the examples also demonstrate that the production of neutralizing antibodies against SARS-COV-2 can be achieved with the polypeptides and oligomers of the invention.

In a further aspect, the invention relates to an immunogenic composition or a vaccine comprising the polypeptide, the polynucleotide or the vector according to the invention and a pharmaceutically suitable carrier or excipient.

The term "vaccine", as used herein, refers to a formulation designed to induce an immune response against an antigen. The vaccine may be therapeutic, which is administered during treatment to boost the immune response or drive a response in a particular direction; or may be prophylactic, which is administered before or shortly after developing a disease. The vaccine may be both therapeutic and prophylactic at the same time in treating disease that has developed and preventing disease that will recur in the future. The vaccine can be administered to a subject by conventional administration methods in the art. As used herein, the term "administration" or "administering" includes all suitable means of providing a substance to a patient. Conventional routes include administration by oral, sublingual, transmucosal, transdermal, rectal, vaginal, subcutaneous, intramuscular, intravenous, intraarterial, intrathecal, administration via catheters, and administration via implants, etc.

In one preferred embodiment, the immunogenic composition or vaccine can be used in the prevention or treatment of a disease caused by the infection of SARS-CoV-2. Preferably, the vaccine is administrated in two doses 15 to 30 days apart.

### Methods for the detection of anti-SASR-CoV2 antibodies and for the detection of infection by SARS-CoV2:

The authors of the invention have found that the polypeptides and trimers of the invention provide a reliable target for detection of antibodies specific for the spike protein of SARS-CoV-2 in a sample. If the sample is obtained from a subject, presence or absence of immunity against SARS-CoV-2 in said subject can be efficiently determined based on the presence or absence, respectively, of neutralizing or no-neutralizing antibodies against the S protein in the sample. The method also contemplates the detection of the presence of antibodies in samples which are not obtained from a subject, for example but not limited to, antibodies produced *in vitro* using cell lines, such as hibridomas.

Thus, in a further aspect, the invention relates to a method for the detection of the presence of antibodies specific for the SARS-CoV-2 spike protein in a sample comprising the steps of:
(i) contacting the sample with polypeptide or the trimeric polypeptide according to the invention and,
(ii) detecting the formation of a complex between antibodies specific for the SARS-CoV-2 spike protein present in the sample and the polypeptide or trimeric polypeptide of step (i).

In one embodiment, the detection step (ii) is carried after the complexes containing the polypeptide or trimeric polypeptide and the antibodies specific for the SARS-CoV-2 spike protein are separated from the remaining components of the sample.

In a further preferred embodiment, the polypeptide or the trimeric polypeptide is immobilized.

In another aspect, the invention relates to a method for detecting whether a subject has suffered an infection by SARS-CoV-2 comprising the detection of antibodies against the SARS-CoV-2 spike protein in a sample of the subject by a method as defined in any of the previous methods.

The term "subject" or "patient" is meant any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on.

The term "specific for" or "specifically binds" when referring to antibodies and antigen binding fragments thereof means that the antibody binds to the polypeptides and oligomers of the invention or variants thereof with no or insignificant binding to other proteins. The term however does not exclude the fact that antibodies may also be cross-reactive with other forms of the polypeptides or oligomers of the invention. Typically, the antibody binds with an association constant (Kd) of at least about 1 × 10⁻⁶ M or 10⁻⁷ M, or about 10⁻⁸ M to 10⁻⁹ M, or about 10⁻¹⁰ M to 10⁻¹¹ M or higher, and binds to the predetermined antigen with an affinity that is at least two-fold greater than its affinity for binding to a non-specific antigen (e.g., BSA, casein) other than the predetermined antigen or a closely-related antigen. The phrases "an antibody recognizing an antigen" and "an antibody specific for an antigenic protein" are used interchangeably herein with the term "an antibody which binds specifically to an antigenic protein".

The phrase "specific for" or "specifically bind(s)" or "bind(s) specifically" when referring to a polypeptide refers to a polypeptide molecule which has intermediate or high binding affinity, exclusively or predominately, to a target molecule. The phrase "specifically binds to" refers to a binding reaction that is determinative of the presence of a target protein in the presence of a heterogeneous population of proteins and other biologics. Thus, under designated assay conditions, the specified binding moieties bind preferentially to a particular target protein and do not bind in a significant amount to other components present in a test sample. Specific binding to a target protein under such conditions may require a binding moiety that is selected for its specificity for a particular target antigen. A variety of assay formats may be used to select ligands that are specifically reactive with a particular protein. For example, solid-phase ELISA immunoassays, immunoprecipitation, Biacore, and Western blot are used to identify antibodies that specifically react with the polypeptides and oligomers of the invention or their variants thereof. Typically, a specific or selective reaction will be at least twice background signal or noise and more typically more than 10 times background.

### Method for generating antibodies comprising the use of the polypeptide according to the invention.

In a further aspect, the invention relates to a method for producing a population of antibodies against SARS-CoV-2, said method comprising the steps of:
(i) administering the polypeptide or the trimeric polypeptide according to the invention into a subject so that an immune response against said polypeptides or oligomers is elicited in said subject and;
(ii) isolating from said subject antibodies, antibody fragments and/or antibody-producing cells recognizing the polypeptides according to the invention.

An "antibody" is used in the broadest sense and specifically covers polyclonal antibodies and antibody fragments so long as they exhibit the desired biological activity. In particular, an antibody is a protein including at least one or two, heavy (H) chain variable regions (abbreviated herein as VHC), and at least one or two light (L) chain variable regions (abbreviated herein as VLC). The VHC and VLC regions can be further subdivided into regions of hypervariability, termed "complementarity determining regions" ("CDR"), interspersed with regions that are more conserved, termed "framework regions" (FR). The extent of the framework region and CDRs has been precisely defined using either the Kabat definition or the Chothia definition. Preferably, each VHC and VLC is composed of three CDRs and four FRs, arranged from amino-terminus to carboxy-terminus in the following order: FRI, CDRI, FR2, CDR2, FR3, CDR3, FR4.

The VHC or VLC chain of the antibody can further include all or part of a heavy or light chain constant region. In one embodiment, the antibody is a tetramer of two heavy immunoglobulin chains and two light immunoglobulin chains, wherein the heavy and light immunoglobulin chains are inter-connected by, e.g., disulfide bonds. The heavy chain constant region includes three domains, CHI, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system. The term "antibody" includes intact immunoglobulins of types IgA, IgG, IgE, IgD, IgM (as well as subtypes thereof), wherein the light chains of the immunoglobulin may be of types kappa or lambda.

The term antibody, as used herein, also refers to a portion of an antibody that binds to the SARS-CoV-2 spike protein e.g., a molecule in which one or more immunoglobulin chains is not full length, but which binds to the SARS-CoV-2 spike protein. Examples of binding portions encompassed within the term antibody include (i) a Fab fragment, a monovalent fragment consisting of the VLC, VHC, CL and CHI domains; (ii) a F(ab')₂ fragment, a bivalent fragment comprising two Fab fragments linked by a disulfide bridge at the hinge region; (iii) a Fc fragment consisting of the VHC and CHI domains; (iv) a Fv fragment consisting of the VLC and VHC domains of a single arm of an antibody, (v) a dAb fragment, which consists of a VHC domain; and (vi) an isolated complementarity determining region (CDR) having sufficient framework to bind, e.g. an antigen binding portion of a variable region. An antigen binding portion of a light chain variable region and an antigen binding portion of a heavy chain variable region, e.g., the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv). Such single chain antibodies are also encompassed within the term antibody. These are obtained using conventional techniques known to those with skill in the art, and the portions are screened for utility in the same manner as are intact antibodies.

In a first step, the method for producing antibodies according to the present invention comprises the administering the polypeptides or trimers according to the invention into a subject so that an immune response against said polypeptides or trimers is elicited in said subject. It will be understood that the administration step can be carried out using the polypeptides or trimers according to the present invention as well as using cells which have been genetically modified so as to express the polypeptides or trimers.

The polypeptides and trimers according to the invention are used as immunogens separately or in combination, either concurrently or sequentially, in order to produce antibodies specific for the SARS-CoV-2 spike protein. Non-limiting examples of suitable adjuvants for animal/veterinary use include Freund's (complete and incomplete forms), alum (aluminium phosphate or aluminium hydroxide), saponin and its purified component Quil A.

The antibodies may be either monoclonal or polyclonal produced using standard techniques well known in the art. See Harlow E, Lane D, "Antibodies: A 25 Laboratory Manual" (Cold Spring Harbor Laboratory Press, Cold Spring Harbor, NY, USA, 1988). Methods for generating other types of antibodies, such as nanobodies, chimeric antibodies and the like, are well known in the art and they are incorporated by reference herein.

In a second step, the method for producing antibodies according to the present invention comprises isolating from said subject antibodies, antibody fragments and/or antibody-producing cells recognizing the polypeptides or trimers according to the invention.

In one instance, the antibody produced herein is further purified to obtain preparations that are substantially homogeneous for further assays and uses. Standard protein purification methods known in the art can be employed. The following procedures are exemplary of suitable purification procedures: fractionation on immunoaffinity or ionexchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75. In one embodiment, protein A immobilized on a solid phase is used for immunoaffinity purification of the antibody products of the invention.

The invention is defined by means of the following aspects:
1. A polypeptide comprising a region from a SARS-CoV-2 spike protein containing at least one mutation selected from the group consisting of: V987H, L849K, S758E, K947R, Q755R, T961E, V963E, D985L, S982F, L517Y, G416R, K386R, V987W, D427I, D420R, A372W and D420Y, relative to the SARS-CoV-2 spike protein sequence as defined in SEQ ID NO: 77, or a functionally equivalent or immunogenically active variant thereof.
2. The polypeptide according to aspect 1 comprising one of the following sets of mutations:
   - A372W and D420R;
   - D198F, G232L, A372W, N394Q and D420R
   - Q755K, L849K, A892N, K947R and K1045R and
   - S758E, T912R and K947R.
3. The polypeptide according to aspect 1 comprising one of the following set of mutations:
   - A706Q, S758E, L849K, V963E, S1030R and Q1113R;
   - S758E, L849K and V963E;
   - Q755K, C851Q and T961E;
   - A706Q, S1030R and Q1113R; and
   - S758E, T912R and K947R.
4. The polypeptide according to aspect 1, wherein the polypeptide comprises:
   - the V987H mutation and a K986P mutation relative to the SARS-CoV-2 spike protein sequence as defined in SEQ ID NO:77
   - The L849K mutation and the K986P and V987P mutations relative to the SARS-CoV-2 spike protein sequence as defined in SEQ ID NO:77 and
   - The S758E mutation and the K986P and V987P mutations relative to the SARS-CoV-2 spike protein sequence as defined in SEQ ID NO:77
   or the polypeptide according to aspect 3 which further comprises the K986P and V987P mutations relative to the SARS-CoV-2 spike protein sequence as defined in SEQ ID NO: 77.
5. The polypeptide according to any of aspects 1 to 4 wherein the naturally occurring furin cleavage site in the SARS-CoV-2 spike protein has been mutated to avoid said site being recognized by a protease.
6. The polypeptide according to aspect 5 wherein the naturally occurring furin cleavage site is has been mutated to the GSASS (SEQ ID NO:72) sequence.
7. The polypeptide according to any of aspects 1 to 6 comprising a sequence selected from the group consisting of SEQ ID NO:1 to 21.
8. The polypeptide according to any of aspects 1 to 7 further comprising a trimerization motif located C-terminally.
9. The polypeptide according to aspect 8 wherein the trimerization motif comprises SEQ ID NO: 22.
10. The polypeptide according to any of aspects 1 to 9 further comprising an affinity tag.
11. The polypeptide according to aspect 10 wherein the affinity tag is a histidine rich region, preferably an hexahistidine region (SEQ ID NO;23), and/or a Streptag II (SEQ ID NO:24).
12. The polypeptide according to aspect 10 or 11 wherein if the polypeptide comprises a trimerization motif, the affinity tag is located C-terminally with respect to trimerization motif.
13. The polypeptide according to any of aspects 10 to 12 further comprising a target site for a protease.
14. The polypeptide according to aspect 11 wherein the target site for a protease is a target site for the HRV C3 protease having the sequence of SEQ ID NO:26.
15. The polypeptide according to aspects 13 or 14 wherein if the polypeptide comprises a trimerization motif and an affinity tag, then the target site for a protease is located in between the trimerization motif and the affinity tag.
16. The polypeptide according to any of aspects 1 to 15 wherein the polypeptide does not contain the transmembrane region (TM) and/or the C-terminal tail (CT) of the SARS-CoV-2 spike protein.
17. The polypeptide according to any of aspects 1 to 16 comprising a sequence selected from the group consisting of SEQ ID NO:28 to 47.
18. A trimeric polypeptide which is formed by three monomer polypeptides, wherein each monomer polypeptide is as defined in any of aspects 1 to 17 and wherein each of the monomer polypeptides comprises a trimerization motif.
19. A polynucleotide encoding the polypeptide of any of aspects 1 to 18.
20. The polynucleotide according to aspect 19, wherein the polynucleotide further comprises a sequence encoding a signal peptide in the same open reading frame as the polypeptide.
21. The polynucleotide according to aspect 20, wherein the sequence encoding a signal peptide is selected from SEQ ID NO:48 or SEQ ID NO:49.
22. The polynucleotide according to any of aspects 19 to 21 wherein the polynucleotide comprises a sequence optimized for expression in mammalian cells.
23. The polynucleotide according to any of aspects 19 to 22 which comprises the sequences according to SEQ ID NO:50 to 70.
24. A vector comprising the polynucleotide according to any of aspects 19 to 23.
25. A host cell comprising the vector according to aspect 24.
26. A method of producing a polypeptide according to any of aspects 1 to 17, comprising the steps of:
   (i) expressing in a suitable expression system a polynucleotide according to any of aspects 19 to 23 and;
   (ii) recovering the polypeptides from the expression system.
27. The method according to aspect 26 wherein if the polypeptide comprises a trimerization motif, then the expression is carried out under conditions adequate for allowing the trimerization of said polypeptides, so that trimer polypeptides are recovered in step (ii).
28. The method of aspect 27 wherein the trimeric polypeptides are contacted with a protease which is capable of recognizing and cleaving the target site found in the monomers, thereby producing trimeric polypeptides devoid of the affinity tags.
29. A method according to aspect 26 to 28 wherein if the polynucleotide comprises a sequence encoding a signal peptide then the polypeptide is recovered from the supernatant of the expression system.
30. A method according to aspect 26 to 29 wherein the polypeptides are purified with a ligand with the capacity to bind to the affinity tag.
31. A method according to any of aspects 26 to 30 wherein the expression system is a mammalian cell line culture.
32. A polynucleotide according to any of aspects 1 to 17, a trimeric polypeptide according to aspect 18, a polynucleotide according to any of aspects 19 to 23, or a vector according to aspect 24 for use in the prevention or treatment of a disease caused by the infection by SARS-CoV-2.
33. An immunogenic composition or a vaccine comprising the polypeptide according to any of aspects 1 to 17, the trimeric polypeptide according to aspect 18, the polynucleotide according to any of aspects 19 to 23, or the vector according to aspect 24 and a pharmaceutically suitable carrier or excipient.
34. The immunogenic composition according for aspect 33 for use in the prevention or treatment of a disease caused by the infection by SARS-CoV-2.
35. The immunogenic composition for use according to aspect 34, wherein the vaccine is administrated in two doses 15 to 30 days apart.
36. A method for the detection of the presence of antibodies specific for the SARS-CoV-2 spike protein in a sample comprising the steps of:
   (i) contacting the sample with polypeptide according to any of aspects 1 to 17 or the trimeric polypeptide according to aspect 18 and,
   (ii) detecting the formation of a complex between antibodies specific for the SARS-CoV-2 spike protein present in the sample and the polypeptide or trimeric polypeptide of step (i).
37. The method according to aspect 36, wherein the detection step (ii) is carried after the complexes containing the polypeptide or trimeric polypeptide and the antibodies specific for the SARS-CoV-2 spike protein are separated from the remaining components of the sample.
38. The method according to aspects 36 or 37, wherein the polypeptide or the trimeric polypeptide is immobilized.
39. A method for detecting whether a subject has suffered an infection by SARS-CoV-2 comprising the detection of antibodies against the SARS-CoV-2 spike protein in a sample of the subject by a method as defined in any of aspects 36 to 38.
40. A method for producing a population of antibodies against SARS-CoV-2, said method comprising the steps of:
   (i) administering the polypeptide according to any of aspects 1 to 17 or the trimeric polypeptide according to aspect 18, into a subject so that an immune response against said polypeptides or oligomers is elicited in said subject and;
   (ii) isolating from said subject antibodies, antibody fragments and/or antibody-producing cells recognizing the polypeptides of step (i).

### EXAMPLES

### Materials and Methods

*Recombinant trimeric Spike glycoprotein design and modelling.*

Unsolved secondary structures of the trimer in closed (PDB: 6VXX) and open (PDB: 6VYB) conformations⁸ (Walls, A. C. *et al.* Cell 1-12 (2020)) were reconstructed using SwissModel³¹ (Waterhouse, A. *et al.* Nucleic Acids Res 46, W296-W303 (2018)). Then, all possible single mutations in both conformations were modelled using FoldX³² (Schymkowitz, J. *et al.* Nucleic Acids Res 33, (2005)). Comparison of the Gibbs free energy changes upon mutation between the open (ΔΔGopen) and closed (ΔΔGclosed) conformations (ΔΔG) revealed a set of mutations predicted to strengthen the closed conformation. All single mutations predicted with ΔΔG < -1 kcal/mol were rationally addressed by inspecting the three-dimensional models. In this regard, the final selection was based in two steps: i) selection of mutations predicted to increase the stability of the closed-conformation over the open-conformation using FoldX, ii) selection of mutations creating well-defined intermolecular interactions between the RBDs (including hydrophobic bonds, π-π interactions and cation-π interactions, ionic bonds, hydrophobic contacts or cavity filling mutations) that would exert a negative impact on the opening motion of the trimer.

*Recombinant protein production and purification.*

The design of recombinant Spike glycoprotein is based on the one described by (Wrapp, D. et al. Science. 367, 1260-1263 (2020)). Briefly, the C-terminal end of the extracellular portion of the Spike was fused to a T4-foldon trimerization domain followed by an 8xHis tag and strep tag II. Plasmids for the expression of the recombinant Spike glycoproteins were obtained from GeneART (ThermoFisher scientific) in a pcDNA3.4 backbone. Proteins were produced by transient transfection using the ExpiFectamine 293 transfection kit (ThermoFisher Scientific) and following manufacturer specifications. Cell culture supernatants were harvested five days after transfection, clarified by centrifugation (3000xg for 20 minutes) or using Sartoclear Dynamics^{®} Lab V (Sartorius) and filtered at 0.2 µm using Nalgene Rapid-Flow sterile single use vacuum filter units (ThermoFisher Scientific). Equivalent transfection efficiency was obtained for all the tested Spike variants. Proteins were purified by Immobilized Metal Affinity Chromatography using the Ni-Sepharose Excel histidine-tagged protein purification resin (Cytiva). Purified proteins where concentrated and buffer exchanged to phosphate buffer saline by ultrafiltration (Merck Millipore) and stored at -80°C until use. Purified proteins were quantified by ELISA. Briefly, Nunc MaxiSorp ELISA plates were coated overnight at 4°C with 100 ng/well of HIS.H8, an anti-6xHis monoclonal antibody, in PBS (ThermoFisher Scientific). The following day, plates were blocked with PBS/1% of bovine serum albumin (BSA, Miltenyi BiotecBiotec) for two hours at room temperature. Commercial His-Spike (Sino Biological) was used as standard, starting at 1µg/mL and eight 1/3 serial dilutions. Spike variants were prepared in blocking buffer at 1/10 serial dilution for quantification. Samples were incubated overnight at 4°C. After that, plates were washed and incubated for 2 hours at room temperature with rabbit anti-SARS-CoV-2 Spike S2 IgG (SinoBiological) at 1/1000 dilution. Then, the HRP conjugated donkey anti-rabbit IgG (H+L) antibody (Jackson ImmunoResearch) at 1/10000 dilution was used as detection antibody. Plates were revealed with o-Phenylenediamine dihydrochloride (OPD) (Sigma-Aldrich) and stopped using 4N of H₂SO₄ (Sigma- Aldrich). The signal was analyzed as the optical density (OD) at 492 nm with noise correction at 620 nm.

Integrity and purity of purified proteins were analyzed by sodium dodecyl sulfate polyacrylamide gel electrophoresis (SDS-PAGE) and Coomassie G-250 staining (ThermoFisher Scientific).

*Cell culture, viral isolation and titration.*

Vero E6 cells (ATCC CRL-1586) were cultured in Dulbecco's modified Eagle medium (Invitrogen) supplemented with 10% fetal bovine serum (FBS; Invitrogen), 100 U/mL penicillin, 100 µg/mL streptomycin, (all from Invitrogen).

Both SARS-CoV-2 isolates used in the present work (Cat 01 and Cat 24) have been described previously (Perez-Zsolt, D. et al. Cell Mol Immunol 18, 2676-2678 (2021); Rodon, J. et al. Front Pharmacol 12, 1-12 (2021)) and were isolated from a clinical nasopharyngeal swab, as previously described in (Rodon, J. et al. Front Pharmacol 12, 1-12 (2021)). Viral isolates were subsequently grown in Vero E6 cells. Cat 01 is a SARS-CoV-2 D614G variant (ID EPI_ISL_510689) whereas Cat 24 is a SAR-CoV-2 B.1.351 variant (originally detected in South Africa; EPI_ISL_1663571). Genomic sequencing was performed from viral supernatant by using standard ARTIC v3 or v4 based protocols followed by Illumina sequencing (dx.doi.org/10.17504/protocols.io.bhjgj4jw). Raw data analysis was performed by viralrecon pipeline (https://github.com/nf-core/viralrecon) while consensus sequence was called using samtools/ivar at the 75% frequency threshold. SARS-CoV-2 D614G variant (ID EPI_ISL 510689) and SARS-CoV-2 B.1.351 variant (originally detected in South Africa; EPI_ISL_1663571) sequences were deposit at GISAID Viral stock was titrated in 10-fold serial dilutions on Vero E6 cells to calculate the TCID50 per mL.

*In vivo immunization and challenge experiments.*

All animal procedures were performed under the approval of the Committee on the Ethics of Animal Experimentation of the IGTP and the authorization of Generalitat de Catalunya (Code: 10965 and 11094).

Prophylactic activity of the recombinant S-2P, S-V987H trimeric proteins and a recombinant monomeric RBD against SARS-CoV-2 D614G isolate (Cat01 isolate) was assessed in B6. Cg-Tg(K18-ACE2)2Prlmn/J (K18-hACE2) mice (stock #034860, Jackson Laboratories) and Golden Syrian hamsters (GHS) (Envigo). In addition, protection against the SARS-CoV-2 B.1.351 (Beta) isolate (Cat024 isolate) was evaluated in K18-hACE2 mice. The colony of these mice was maintained by breeding K18-hACE2 hemizygotes with C57BU6J mice following the instructions of Jackson Laboratory (https://www.jax.org/strain/034860). Mice genotyping was performed according to the protocol 38170: Probe Assay - Tg(K18-ACE2)2Prlmn QPCR version 2.0 (https://www.jax.org/Protocol?stockNumber=034860&protocolID=38170). The colony of GSH was maintained by brother/sister mating. Both mice and GSH colonies were stablished at the Centre for Comparative Medicine and Bioimage (CMCiB).

For the SARS-CoV-2 D614G challenge, Sixty-eight K18-hACE2 mice (50%male/50% female, 7-9 weeks old) were distributed in five experimental groups: S-2P (n=16), V987H (n=14), RBD (n=15), infected positive controls (n=19) and unvaccinated/uninfected negative control (n=4). Sixty-eight GSH (were distributed in S-2P, S-V987H, RBD and infected positive controls (n=16/group) and unvaccinated/uninfected negative control (n=4). Mice and GSH from the S-2P, S-V987H and RBD groups were DNA-immunized by electroporation in the quadricep posterior. Forty microgram of plasmid coding for the corresponding immunogens were used. Animals were electroporated using a NEPA21 electroporator and tweezer electrodes (Nepagene). Two (for mice) or four (for GSH) weeks later, DNA-immunized animals received a boosting dose consisting of 15 µg of recombinant protein adjuvanted with adjust-Phos (Invivogen) in the hock (Kamala, T. J Immunol Methods 328, 204-214 (2007)). Control animals were primed with an empty vector and boosted with PBS+Adjust-Phos. Two weeks (mice) or ten days (GSH) after boosting, animals were intranasally challenged with 10000 TCID₅₀ of SARS-CoV-2 (Cat01 isolate) and followed up for 7 days. Weight and clinical signs were monitored daily after infection. Four animals for each experimental group, except for uninfected controls, were euthanized before challenge and on days 2, 4 or 7 post infection. Uninfected controls were euthanized on day 7 post-infection and only two mice from groups S-V987H and RBD were euthanized before challenge. However, any animal that showed a reduction of weight higher than 20%, a drastic reduction of mobility or a significant reduction of the response to stimuli were euthanized according to the humane endpoints defined in the supervision protocol. After euthanasia, oropharyngeal swab, nasal turbinate, lung and brain were collected for viral load determination and histopathological analysis. Blood samples were collected before each immunization and viral challenge, and at euthanasia. Blood was left at room temperature for two hours for clotting and serum was collected after centrifugation (10 minutes at 5000xg) and stored at -80°C until use.

For the SARS-CoV-2 B.1.351 (Beta) VoC challenge, Sixty-eight K18-hACE2 mice (50% male) were distributed as follows: S-2P (n=21), S-V987H (n=21), infected positive controls (n=16) and unchallenged controls (n=10). Mice from S-2P and S-V987H groups were immunized twice (spaced three weeks between both doses) with 15 µg of recombinant protein adjuvanted with AddaVax (Invivogen) in the hock. Control mice received only PBS+AddaVax. Two weeks after the booster, mice from groups S-2P, S-V987H and challenged-controls were inoculated intranasally with 1000 TCID50 of SARS-CoV-2 Beta VoC (Cat24 isolate).

In a second set of experiments, ninety-one K18-hACE2 mice (50% female and 50% male, age 7-9 weeks) were distributed in five experimental groups: S-2P (n=21), S-21(n=22), S-29 (n=22), unvaccinated and challenged controls (n=16), and uninfected negative controls (n=10). In the case of GSHs, a total of forty-nine animals were used (1:1 female-male ratio, age 5-7 weeks) and distributed in five experimental groups: S-2P (n=11), S-21 (n=11), S-29 (n=11), unvaccinated and challenged controls (n=11), and uninfected negative controls (n=54). Both mice and hamsters from S-2P, S-21, and S-29 groups were immunized with 15 µg of recombinant protein with AddaVax (Invivogen) as adjuvant in the hock (Kamala, T. J Immunol Methods 328, 204-214 (2007)). Three weeks later, immunized animals were boosted with a second dose of the same formulation. Control animals were primed and boosted with PBS and Addvax. Two weeks after boosting, mice were challenged with 1000 TICD50 of SARS-CoV-2 (Cat24 isolate), and follow up for 14 days. GSH were challenged three weeks after boosting with 10000 TICD50 of SARS-CoV-2 (Cat24 isolate) and follow up for 7 days. Animals were monitored daily after infection, and weight and clinical signs recorded daily. Six mice for each experimental group, except the uninfected controls, were euthanized on days 3 and 6. The remaining mice were followed up until day 14 post-infection. Three and four hamsters from each experimental group, except the uninfected control group, were euthanized on days 2 and 4, respectively. The remaining GSHs were euthanized on day 7 post infection. In both challenge experiments, uninfected control group was euthanized at the end of the experiment. In addition, any animal showing weight loss higher than 20%, a drastic lack of motility, or a significant reduction of their response to stimuli were euthanized according to the humane endpoints defined in the supervision protocol. Biological samples were collected after euthanasia, including oropharyngeal swab, nasal turbinate, lung, and brain (only in the case of mice) to determine viral loads and perform histopathological analysis. Furthermore, blood samples were acquired before each immunization, viral challenge, and under euthanasia. Blood was left at room temperature for two hours for clotting, and serum was collected after centrifugation (10 minutes at 5000xg) and stored at -80°C until use.

*Quantification of anti-Spike antibodies by ELISA.*

IgG antibodies elicited against the Spike and RBD glycoproteins were determined using and in-house ELISA in serum samples obtained from animals before each immunization and before viral challenge. In addition, humoral response was also evaluated in serum samples obtained from animals euthanized on days 2, 4, 7 after viral challenge or after humane endpoint. One half of a Nunc MaxiSorp ELISA plate was coated overnight at 4°C with 50 ng/well of antigen in PBS (Spike or RBD, Sino Biologicals). The other half-plate was incubated only with PBS. Then, the whole plate was blocked using PBS/1% of bovine serum albumin (BSA, Miltenyi Biotech) for two hours at room temperature. Mouse standards were prepared as seven 1/3 dilution of the anti-6xHis antibody HIS.H8 (ThermoFisher Scientific), starting at 1 µg/mL. GSH standard was prepared similarly but using a positive GSH serum with the initial dilution at 1/100. All standards and samples were diluted in blocking buffer. After blocking, 50 µL of each standard or diluted samples were added to the antigen coated and antigen free wells in duplicate and incubated overnight at 4°C. Each plate contained samples from all experimental groups. Plates were run in parallel to reduce inter-assay variability. After sample addition, plates were incubated overnight at 4°C. The HRP conjugated (Fab)2 Goat anti-mouse IgG (Fc specific) (1/20,000), or Goat anti-hamster IgG (H+L) (1/20,000) (all from Jackson Immuno Research) were used as detection antibodies for mouse and GSH IgG determination, respectively. Plates were revealed with o-Phenylenediamine dihydrochloride (OPD) (Sigma-Aldrich) and stopped using 2N or 4N of H₂SO₄ (Sigma-Aldrich). The signal was analyzed as the optical density (OD) at 492 nm with noise correction at 620 nm.

The specific signal for each sample was calculated after subtracting the background signal obtained in antigen-free wells. Data is shown as arbitrary units (AU) according to the standard used.

*Neutralizing activity of serum samples.*

The neutralizing activity of serum samples was determined using HIV reporter pseudoviruses expressing SARS-CoV-2 S protein and Luciferase as described by Pradenas *et al.* (Pradenas, E. et al. Med 135907 (2021)). In brief, pseudoviruses were produced by co-transfecting Expi293F cells (ThermoFisher Scientific) with the pNL4-3.Luc.R-.E- (NIH AIDS Reagent Program (Connor, R. I., Chen, B. K., Choe, S. & Landau, N. R. Virology vol. 206 935-944 (1995)). and several SARS-CoV-2.SctΔ19 plasmids that code for the Spike glycoprotein of the WH1, Beta, Delta or Omicron variants. A VSV-G plasmid was used for the generation of VSV-G-pseudoviruses that were used as negative control. Transfections were performed using the ExpiFectamine293 Reagent kit (ThermoFisher Scientific). After 48h, supernatants were harvested, filtered at 0.45 µm and frozen at -80°C until use. Pseudoviruses were titrated on HEK293T cells overexpressing WT human ACE-2 (HEK293T/hACE2) (Integral Molecular).

Serum samples were inactivated at 56°C for 60 minutes before use. Inactivated samples were 1/3 serially diluted in cell culture medium (DMEN, 10% fetal bovine serum) (range 1/100-1/24300) before mixing with 200 TCID50 of SARS-CoV-2 derived pseudoviruses and incubated for 1 hour at 37°C. Then, 2×10⁴ HEK293T/hACE2 cells treated with DEAE-Dextran (Sigma-Aldrich) were added. After 48 hours BriteLite Plus Luciferase reagent (PerkinElmer) was added and the results read in an EnSight Multimode Plate Reader. Data were calculated using a 4-parameters logistic equation in Prism 8.4.3 (GraphPad Software) and showed as normalized ID50 (reciprocal dilution inhibiting 50% of the infection). This assay has been previously validated with a replicative viral inhibition assay (Trinité, B. et al. Sci Rep In press, 1-10 (2021)).

*Viral load quantification in oropharyngeal swab and tissue samples.*

Oropharyngeal swabs and samples from nasal turbinate, lung and brain (only mice) were collected immediately after euthanasia in 1.5 mL Sarstedt tubes containing DMEM media supplemented with penicillin (100 U/mL) and streptomycin (100 µg/mL). Tissue samples were homogenized twice at 25 Hz for 30 sec using a TissueLyser II and a 1.5 mm Tungsten bead (QIAGEN). After centrifugation for 2 min at 2,000 × g, supernatants were collected and stored at -80 °C until use.

RNA was isolated using the Viral RNA/Pathogen Nucleic Acid Isolation kit and a KingFisher instrument (ThermoFisher Scientific), or an IndiMag pathogen kit (Indical Bioscience) on a Biosprint 96 workstation (QIAGEN) following manufacturer's instructions.

PCR amplification in mice was based on the 2019-Novel Coronavirus Real-Time RT-PCR Diagnostic Panel guidelines and protocol developed by the American Center for Disease Control and Prevention. Briefly, a 20 µL PCR reaction was set up containing 5 µL of RNA, 1.5 µL of N2 primers and probe (2019-nCov CDC EUA Kit, Integrated DNA Technologies) and 10 µl of GoTaq 1-Step RT-qPCR (Promega, Madison). Thermal cycling was performed at 50°C for 15min for reverse transcription, followed by 95°C for 2 min and then 45 cycles of 95°C for 10 sec, 56°C for 15 sec and 72°C for 30 sec in the Applied Biosystems 7500 or QuantStudio5 Real-Time PCR instruments (ThermoFisher Scientific). For absolute quantification, a standard curve was built using 1/5 serial dilutions of a SARS-CoV2 plasmid (2019-nCoV_N_Positive Control, 200 copies/µL, Integrated DNA Technologies) and run in parallel in all PCR determinations. The viral load of each sample was determined in triplicate and mean viral load (in copies/mL) was extrapolated from the standard curve and corrected by the corresponding dilution factor. Alternatively, results are shown as Ct or 2-ΔCt.

SARS-CoV-2 subgenomic RNA (sgRNA) was performed as previously described (Wölfel, R. et al. Nature 581, 465-469 (2020)) with the following primers (Forward; 5-CGATCTCTTGTAGATCTGTTCTC-3'; Reverse, 5'-ATATTGCAGCAGTACGCACACAA-3') and probe (5'- FAM-ACACTAGCCATCCTTACTGCGCTTCG-TAMRA-3').

Mouse gapdh gene expression was measured in duplicate for each sample using TaqMan gene expression assay (ThermoFisher Scientific) as amplification control.

SARS-CoV-2 genomic RNA (gRNA) detection in GHS was performed based on RT-PCR described by Corman *et al.* (Corman, V. M. et al. Eurosurveillance 25, 2000045 (2020)) which was adapted to the AgPath-ID One-Step RT-PCR kit (Life Technologies). This RT-PCR targets a fragment of the envelope protein gene using the following primers (Forward: 5'-ACAGGTACGTTAATAGTTAATAGCGT-3'; Reverse: 5'-ATATTGCAGCAGTACGCACACA-3') and probe (5'-FAM-ACACTAGCCATCCTTA CTGCGCTTCG-TAMRA-3'). Thermal cycling was performed at 55°C for 10 min for revers transcription, followed by 95°C for 3 min and then 45 cycles of 94°C for 15s, 58°C for 30s. SARS-CoV-2 subgenomic RNA detection in GHS was done as it is described in Wölfel *et al.* (Wölfel, R. et al. Nature 581, 465-469 (2020)). The primers and probes are the same as in gRNA determination except for forward primer: 5'-CGATCTCTTGTAGATCTGTTCTC-3'. Thermal cycling for sgRNA was performed at 55°C for 10 min for revers transcription, followed by 95°C for 3 min and the 45 cycles of 95°C for 15 s, 56 °C for 30s. Results are shown as Ct or 2-ΔCt.

*Statistical analysis.*

ELISA binding data and neutralizing activity in each time point were analyzed using Connover Test with multiple comparison correction by FDR. Differences among animals within a particular group along time were analyzed using the Friendman test corrected for multiple comparison using false discovery rate (FDR). Weight variation in SARS-CoV-2 challenged mice over time was analyzed using Kruskal-Wallis corrected by Dunn's test. Severe disease incidence was represented by Kaplan-Meier plots. Statistical analysis was performed against unvaccinated group using Mantel-Cox test. Levels of SARS-CoV-2 gRNA in tissues were analyzed using Peto & Peto left-censored k sample test corrected by FDR. Histopathology analysis was carried out using Asymptotic Generalized Pearson Chi-Squared test with FDR correction. P values are indicated as follows: * p<0.05, ** p<0.01, *** p<0.001, **** p<0.0001. Whereas p values close to statistical significance are shown as number. Statistical analyses were conducted using the R (version 3.6) software environment and GraphPad Prism v8.0.

### Example 1: Identification of Spike glycoprotein mutations that constrain the motility of RBD.

A total of 11 single mutations (A372W, K386R, G416R, D420R, D420Y, D427I, L517Y, S982F, D985L, V987H, and, V987W) were selected. In addition, a double mutation (A372W-D420R) referred as 2M, and a combined quintuple mutation (D198F-G232L-A372W-N394Q-D420R) named 5M were selected. Recombinant mutant proteins were expressed by transient transfection in Expi293F cells and their production was evaluated by ELISA (Fig. 1a). Most variants displayed a substantial decrease in production when compared to the S-2P trimer (Fig. 1a). Then, the exposure of the RBD by ELISA using a Fc fusion protein containing the extracellular portion of the human ACE2 receptor fused to the human IgG1-Fc domain was also analyzed. The results confirmed that most of the variants were in fact promoting a closed trimer conformation (Fig. 1b) as it was predicted by the inventor in silico pipeline. Moreover, variants associated with a reduced exposure of the RBD also resulted in a very low production. In contrast, the V987H mutation promoted the exposure of the RBD (Fig. 1b) and showed higher production than the S-2P protein (2.5-fold). Thus, these results suggest that the RBD exposure may be associated with Spike production levels.

### Example 2: S-V987H trimer vaccination protects K18-hACE2 mice from SARS-CoV-2 infection-associated disease.

It has been described that K986P and V987P mutations stabilize and increase the expression and immunogenicity of the Spike glycoprotein (Wrapp, D. et al. Science (1979) 367, 1260-1263 (2020); Corbett, K. S. et al. Nature 586, 567-571 (2020)). Since the V987H mutation improved Spike trimer production and RBD exposure, the inventor evaluated whether it could impact the Spike antigenicity in vivo. Thus, the immunogenicities and protective capacities of S-2P, S-V987H and the purified RBD (Fig. 2a) after SARS-CoV-2 D614G challenge in K18-hACE2 mice (Fig. 2b) were compared. Forty-five K18-hACE2 mice were immunized using a prime-boost immunization strategy (Fig. 2b). In addition to S-V987H (n=14), the inventor determined the immunogenicity of S-2P (n=16), and a recombinant monomeric RBD protein (n=15). Mice were first immunized by DNA electroporation with 40 µg of plasmid. Two weeks later, animals were boosted with the corresponding purified recombinant protein (15µg) formulated with aluminum phosphate as adjuvant. Prior to challenge, four mice from S-2P and control groups, two mice from S-V987H and three mice from RBD groups were euthanized to collect tissue samples. Then, 12 vaccinated mice for each group, and 16 unvaccinated controls were intranasally challenged with SARS-CoV-2 D614G (Fig. 2b). Four mice (two male/two female) from each group were euthanized on days 2, 4 and 7 (end of the experiment) post-challenge to analyze the humoral immune responses, viral replication in target organs, and tissue damage (Fig. 2b). Mice that developed severe SARS-CoV-2 induced disease and/or showed a weight reduction higher than 20% of the initial weight were euthanized before the end of the experiment (day 7) as a humane end-point. Four additional unvaccinated mice were used as uninfected controls.

The humoral responses elicited against the RBD (Fig. 2c), and the S protein (Fig. 8a) were evaluated before each immunization (days -28 and -14) and viral challenge (day - 2), and in the mice euthanized on days 2, 4 and 7 after infection. At all-time points, mice immunized with S-V987H and S-2P showed similar levels of anti-RBD and anti-Spike IgG antibodies, which were greater than those observed in RBD vaccinated animals (p<0.001, Conover-Iman test) (Fig. 2c and Fig. 8a, b and c). For simplification purposes, challenged animals were grouped as a "post-challenge" group (Fig. 2c). The levels of anti-RBD and anti-Spike IgG antibodies in the S-2P and S-V987H groups increased after boost and viral challenge (p<0.05, Conover-Iman test) (Fig 2c and Fig. 8a), while mice immunized with RBD only presented increased levels of these antibodies after viral challenge (p<0.05, Conover-Iman test), indicating that infection may further boost humoral responses in vaccinated mice (Fig. 2c and Fig. 8a, b and c).

In addition, the level of Nabs against the Wuhan-Hu-1 (WH1) strain and Beta (B.1.351) variant after SARS-CoV-2 challenge were evaluated. Both S-2P or S-V987H mice groups developed equivalent titers of NAbs against SARS-CoV-2 WH1, but significantly higher than those measured in the RBD immunized group (Fig. 2d p<0.05, Conover-Iman test). However, only S-2P and S-V987H vaccinated mice had systemic neutralizing activity against the Beta VoC prior to challenge (Fig. 2e). Despite this, the titers of NAbs against WH1 strain were higher than those targeting the Beta VoC (Fig. 8d).

The progressive increase in the levels of total IgG antibodies and/or NAbs observed in both RBD and control-challenged groups after infection (Fig. 2c-d) also supports the idea of a boosting of the humoral response after virus challenge.

To assess the ability of each immunogen to prevent SARS-CoV-2 infection-associated disease, the weight evolution in all groups as an indicator of disease progression in this model (Vidal, E. et al. Vet Pathol-. 030098582110668 (2021)) was measured. The inventor identified weight reduction on day 5 post-infection in mice belonging to infected-control and RBD groups, which is opposed to S-2P and S-V987H vaccinated groups (p<0.05, Kruskal-Wallis corrected by Dunn's test) (Fig. 2f). On day 6 and 7, all animals from the inoculated-control group (n=4), one out of four S-2P vaccinated mice, and two out of four RBD immunized mice had to be euthanized due to disease development (Fig. 2g; p<0.05 compared to control-infected group, Log-rank test) Mice from the S-V987H group did not show clinical signs of disease during the entire experimental procedure (p<0.01 compared to control-infected group, Log-rank test) (Fig. 2f and Fig. 2g).

In addition to the clinical course, the levels of viral replication by RT-qPCR in four samples were measured (Fig. 3a-d): oropharyngeal swab, nasal turbinate, lung and, brain. Although the levels of genomic RNA (gRNA) in oropharyngeal swabs decreased in all groups over time, only S-V987H vaccinated mice became undetectable on day 7. On the contrary, S-2P and RBD vaccinated mice remained positive (Fig. 3a, p<0.05 Peto & Peto Left-censored k sample test). Similar results were observed in nasal turbinates (Fig. 3b), where gRNA was lower in S-2P and S-V987H groups compared to control-infected mice or the RBD group on days 6-7 after viral challenge, and the S-V987H group showed the lowest values by day 7. Interestingly, low levels of gRNA were detected in lung and brain from S-V987H immunized mice (lung p=0.055, brain p<0.05, Peto & Peto Left-censored k sample test) (Fig. 3c and d), whereas a progressive increase was observed in brains from the infected-control and RBD groups, and in the only S-2P vaccinated mouse that developed disease (Fig. 3c and 3d). Conversely, subgenomic RNA (sgRNA), which indicates active viral replication, was not detected at any time point in lung or brain of mice immunized with any of the Spike trimers, or in oropharyngeal swabs on day 4 and 7, except for the single S2-P mouse that developed illness. (Fig. 8e).

To confirm active viral replication, Nucleoprotein (NP) levels in tissues were analyzed by immunohistochemistry (IHC). NP was detected in lung and brain of both control and RBD groups; and in one animal from S-2P group that developed the disease, but not in S-V987H or disease-free S-2P vaccinated mice after challenge (Fig. 3e, p<0.05 asymptotic generalized Pearson Chi-Squared test corrected for multiple comparison using FDR). Low IHC scores were observed in nasal turbinates on days 2 and 4 with no major differences among study groups (Fig. 3e). Tissue damage was in line with the levels of viral antigens detected by IHC (Fig. 3f). No tissue damage was observed in lung or brain of mice vaccinated with S-2P and S-V987H, except for the S-2-P mouse that became sick (Fig. 3f). A low lesion score was observed at early time points after challenge in nasal turbinate of all infected mice (Fig. 3f).

Overall, the immunogenicity of both S-2P and S-V987H trimers was equivalent in K18-hACE2 mice, and greater than the produced by the monomeric RBD immunogen. However, S-V987H vaccination improved mice protection against SARS-CoV-2 D614G variant over the S-2P immunogen, since all mice in S-V987H group were disease free and showed a faster viral clearance in tissues.

### Example 3: S-V987H trimer vaccination protects golden Syrian hamsters from SARS-CoV-2 infection-associated disease.

To confirm the results obtained in the transgenic mouse model, a second experiment was performed using golden Syrian hamster (GSH). Similar to K18-hACE2 mice, GSH were immunized using a prime-boost strategy, and intranasally challenged with SARS-CoV-2 D614G (Fig. 4a). Animals were monitored until day 7 post-inoculation, since it has been described that animals start spontaneously recovering around a week after viral infection. (Sia, S. F. et al. Nature 583, 834-838 (2020); Brustolin, M. et al. Emerg Microbes Infect 12, 1-36 (2021)).

The magnitude of the humoral responses elicited against the Spike and the RBD by both S-2P and S-V987H trimers was similar and greater than those elicited by the RBD immunogen (Fig. 4b and Fig. 9a). The levels of anti-RBD and anti-Spike IgG antibodies increased after each immunization and after viral challenge (p<0.05, Friedman test) (Fig. 4b and Fig. 9a), confirming the results obtained in K18-hACE2 mice. However, unlike mice, infected-control GSHs rapidly developed an anti-Spike humoral response after challenge, showing similar levels of anti-Spike and anti-RBD antibodies on day 7 to those observed in animals immunized with the RBD protein (Fig. 4b and Fig. 9b). When the neutralizing activity of serum samples was analyzed, the inventor observed that GSHs immunized with S-2P or S-V987H proteins neutralized the WH1 variant and, to a lesser extent, the Beta VoC (Fig. 4c and d). The neutralizing activity against WH1 increased overtime after challenge in all study groups (p<0.05, Conover-Iman test). Neutralization of WH1 was also detected in sera from infected control animals by day 4 after challenge, and their titers rapidly increased, becoming similar to the ones observed in S-V987H and RBD groups, and higher than those observed in S-2P vaccinated animals by day 7 (p<0.05, Conover-Iman test). Intriguingly, despite all groups showed similar titers of NAbs on day 7 after challenge, the levels of anti-RBD and anti-Spike binding antibodies (Fig. 4b and Fig 9. a and b) were higher in the S-2P and S-V987H immunized groups than in infected-controls GSH. These results support that SARS-CoV-2 infection induced a rapid humoral response against SARS-CoV-2 in GSH that may be qualitatively different to the one elicited by immunization.

The clinical course after challenge was then evaluated. Animals in both control and RBD groups showed a progressive weight reduction until day 7 (end of the experiment) indicative of disease progression (% of weight in infected controls= 87.3 ± 3.1; RBD group= 84.4±1.4) (Fig. 4e). Such weight loss was not observed in S-2P (98.9 ± 1.3) or S-V987H (98.76 ± 2.4) vaccinated GSH (p<0.05 Kruskal-Wallis corrected by Dunn's test). Thus, both S trimers generated equivalent protection from disease development in vaccinated GSH (Fig. 4e).

The presence of SARS-CoV-2 was determined by RT-qPCR in oropharyngeal swabs and respiratory tissue samples (nasal turbinate and lung). Brain was not evaluated in GSHs since SARS-CoV-2 does not affect the brain in this animal model (Muñoz-Fontela, C. et al. Nature 586, 509-515 (2020)). Despite the levels of gRNA decreased over time in all analyzed samples, the inventor detected lower gRNA levels in nasal turbinate of both S-2-P and S-V987H groups on day 4 compared to the RBD and infected-control groups (Fig. 5; p<0.05, Peto & Peto Left-censored k sample test). Remarkably, contrary to the RBD or infected-control groups, gRNA was undetectable on day 7 post-infection in S-2-P and S-V987H immunized GSHs (Fig. 5a). No major differences were detected in the levels of gRNA in oropharyngeal swabs among the study groups (Fig. 5a). Interestingly, lower levels of gRNA were detected in lungs of S-2P and S-V987H vaccinated animals than in the RBD and infected-control groups on days 2 and 4 (Fig. 5a). While the inventor did not observe significant differences among groups when sgRNA was analyzed in nasal turbinate or oropharyngeal swabs, the inventor detected lower sgRNA levels in the lungs of S-V987H and S-2P groups compared to RBD and infected-control groups on days 2 and 4 after challenge (Fig. 9c).

Viral RNA detection results were in line with the levels of NP detection by IHC (Fig. 5b). No major differences in NP levels were observed among study groups at any time points in nasal turbinate, becoming undetectable by day 7 (Fig. 5b) (p<0.05, Asymptotic Generalized Pearson Chi-Squared Test). However, lower NP levels were detected in lungs of both S-2P and S-V987H vaccinated groups when compared with RBD and infected controls on days 2, 4 and 7. Interestingly, NP was not detected in lungs on day 7 in S-2P and S-V987H groups (Fig. 5b). All study groups showed a similar lesion degree in nasal turbinate, which decreased by day 7 after challenge (p<0.05). By contrast, a lower tissue damage was observed in lung from S-V987H (on days 4 and 7) and in S-2P (on day 7) groups compared to RBD and infected control groups (p<0.05) (Fig. 5c).

Overall, the inventor's results showed that the immunogenicity and protective efficacy of both S-2P and S-V987H trimers are equivalent in GSHs, and higher than the one conferred by RBD vaccination.

### Example 4: S-V987H trimer vaccination protects K18-hACE2 mice from the SARS-CoV-2 Beta-variant challenge

From the beginning of the COVID-19 pandemic, several SARS-CoV-2 VoC have emerged. These VoC have shown different transmissibility, pathogenic potential and resistance to antibodies previously elicited by vaccination or natural infection (Salehi-Vaziri, M. et al. Archives of Virology vol. 167 327-344)). The results described above have shown that S-V987H-vaccinated animals were protected from COVID-19 development after SARS-CoV-2 D614G strain challenge. Additionally, vaccinated animals showed low sera neutralizing activity against the SARS-CoV-2 Beta variant. Since the Beta VoC is one of the most resistant to antibodies elicited by natural infection and the currently available vaccines (Pradenas, E. et al. Cell Rep Med 100523 (2022)), and also induces severe disease in K18-hACE2 mice (Tarrés-Freixas, F. et al. Front Microbiol 13, (2022), it was evaluated whether the immune responses induced by S-V987H could protect against disease development after challenge with the SARS-CoV-2 Beta variant. Thus, the inventor immunized twenty-one K18-hACE2 mice with S-V987H or S-2P, using AddVax as adjuvant in this homologous prime-boost experiment (Fig. 6a). Unvaccinated mice were used as negative and positive controls of infection. Two weeks after receiving the protein boost, mice were challenged with the SARS-CoV-2 Beta variant (Fig. 6a). Three groups of mice were euthanized on days 3 (n=6), 6 (n=6) and 14 (n=9) after challenge. All mice that developed severe disease after day 3 (10 in the infected-control group and one in the S-2P group) were euthanized before day 14 following the humane end points of the protocol and analyzed separately. Of note, both S-2P and S-V987H recombinant proteins induced similar levels of IgG antibodies against the Spike and the RBD, which increased after each boost and after viral challenge (p<0.05, Conover-Iman test) (Fig.6b and Fig. 10a and b). Interestingly, S-V987H immunized mice showed higher sera neutralizing activity against WH1 (n=6; 15376±9203) (Fig 6c), and the Delta VoC (n=6; 775018403) (Fig. 6d) than mice immunized with the S-2P three days after challenge (n=6; WH1: 2913±3524; Delta: 1505±4773) (WH1: p<0.01; Delta: p=0.055; Conover-Iman test). Neutralizing activity against the Beta VoC increased after challenge (p<0.05, Conover-Iman test) (Fig. 6e). In addition, the inventor identified an increasing trend in sera neutralizing activity against Omicron over time (p=0.055, Conover-Iman test) (Fig. 6f). These differences suggest that the humoral responses elicited after S-2P or S-V987H immunization evolved after challenge with the SARS-CoV-2 Beta variant, increasing neutralizing activity against Beta and Omicron VoC, as well as against Delta in the case of the S-2P group. Interestingly, neutralizing activity against the Beta VoC was detected in control-infected mice at clinical endpoint (Fig. 6e) with little or no cross-neutralization activity with other variants (Fig. 6c, d and f). No statistical differences in neutralizing activity were observed on days 6 and 14 between S-V987H and S-2P groups for any of four SARS-CoV-2 variants evaluated (Fig. 6c-f).

A reduction of body weight associated with disease progression was observed in mice from the infected control group starting on day 2 after challenge compared to mice vaccinated with S-2P and S-V987H (Fig. 6g) (p<0.05, Kruskal-Wallis corrected by Dunn's test). Mice in both Spike-vaccinated groups maintained their weight until day 14 [percentage of weight: S-2P=99±4 (n=9); S-V987H=98±5 (n=10)]. Contrarily to the S-2P and infected control groups, no mice from the S-V987H group (n=10) showed any clinical signs of disease (Fig. 6g and 6h) during the experiment (day 14) (p<0.001, Long rank test).

The analysis of viral load in tissues by RT-qPCR showed that both S-2P and S-V987H vaccinated groups had a progressive decrease in gRNA levels in oropharyngeal swabs and lung over time (Fig. 7a) (p<0.05; Peto & Peto Left-censored k sample test). Interestingly, the S-V987H group displayed lower viral loads in nasal turbinate than S-2P and infected control animals on day 3, and also in oropharyngeal swab compared to the infected controls (Fig. 7a) (p<0.05). However, these differences were not maintained over time and both S-trimer immunized groups showed low but equivalent values of gRNA on day 14 in all analyzed tissues (Fig. 7a). In addition, these groups displayed lower viral load in lung and brain compared to the infected control group at day 3 after challenge (Fig. 7a, p<0.05).

Remarkably, NP was hardly detected in lung and brain from S-2P and S-V987H groups by IHC (Fig. 7b), which was in line with the low levels of gRNA detected in these animals. Despite that, S-2P vaccinated mice showed a higher lesion score in lung at day 14 than the S-V987H group (p<0.01; Asymptotic Generalized Pearson Chi-Squared test) (Fig. 7c), indicating that these mice presented a severe lung damage. Interestingly, both Spike-based immunogens protected from viral dissemination to the brain (Fig. 7).

To summarize, the immunogenicity of both S-2P and S-V987H trimers was similar in K18-hACE2 SARS-CoV-2 Beta-infected mice, although S-V987H promoted the development of higher serum neutralization, which might explain the increase in protection observed in S-V987H vaccinated animals, compared to the S-2P group.

*Strategy for Spike glycoprotein stabilization.*

To increase the Spike stability and immunogenicity, two different approaches were followed: 1) introduction of point mutations in the S2 region of the Spike to increase its stabilization (using the open state as a reference structure), and 2) increase RBD exposure by forcing an open conformation. In this regard, a computational pipeline was envisioned involving the three-dimensional modeling of all possible single mutations in both of these scenarios. Moreover, all single mutations showing a preference in any of these two conditions were visually inspected. Those mutations that clearly generated well-defined interactions (hydrogen bonds, ionic interactions of filling hydrophobic pockets) between the different chains of the S trimer were prioritized.

Spike variants were then produced and evaluated their yields. Based on the protein production levels, these glycoproteins were classified into three different groups (Fig. 11a). Group 1 included those constructs (i.e. S-29 and S-22) that were produced at the highest levels (five-fold compared to the S-2P protein). Group 2 contained S-21, S-24, S-26, S-27, S-30, and S-31, whose production was intermediate (two-fold higher than S-2P). Last, Group 3 included those Spike variants with a protein yield lower than S-2P (S-20, S-23, S-25, S-28, S-32, S-33, S-34, S-35, S-36, S-37, and S-38). Remarkably, all constructs designed to increase RBD exposure were in the Group 3, suggesting that those mutations drastically impacted on the Spike stability and/or its production (Fig. 11).

However, most constructs with an improved production, also tended to show a better RBD exposure.

When inspecting the mutants and their potential role in increasing the stabilization of the trimer and/or the exposure of the RBD, interesting patterns were found. For example, variants S22 and S29, with higher expression yield, introduce a positive charge in a local area where three glutamic acid residues, a Glu1092 from each chain, might introduce destabilization. Similarly, the Q1113R mutant in S22 introduces an arginine next to Glu1092. Analogous observations can be extracted of most variants introducing a net charge. We also observe that most variants increasing RBD exposure, such as S21, S24 and S29, introduce the S758E mutation. This mutation is in the vicinity of the tip of the closed RBD domain, where we find the presence of two consecutive aspartic residues, Asp427 and Asp428. Modeling the possible positioning of Glu758 (with an initial significant clash with a helix backbone) we speculate that it will be displaced towards the tip of the RBD domain and destabilize the closed conformation by electrostatic repulsion. Interestingly, S-22 and S-29 constructs shared the conservative mutation K947R located in the middle of the heptad repeat 1 (HR1) helix, which could enhance the thermal stability of the protein.

### Example 5: S-21 and S-29 vaccination protects K18-hACE2 mice from SARS-CoV-2-induced disease

To determine whether S-21 and S-29 protect from SARS-CoV-2-induced disease, an immunization and challenge study using K18-hACE2 mice and SARS-CoV-2 B.1.135 (Beta) VoC was performed (Fig 12a). This experimental setting was used for the following reasons: 1) K18-hACE2 mice develop a severe form of the disease that led to the death of the animal unless they are vaccine-protected; 2) the SARS-CoV-2 Beta variant is partially resistant to antibodies elicited by natural infection or vaccination with immunogen based on the original strain (Wuhan, WH1); and 3) the SARS-CoV-2 Beta variant is more pathogenic than the original SARS-CoV-2 strain (WH1) in K18-hACE2 mice 17. Thus, five experimental groups were established: S-2P (n=21), S-21(n=22), S-29 (n=22), infected positive controls (n=16), and uninfected negative controls (n=10). Mice from S-2P, S-21, and S-29 groups were immunized twice, three weeks apart. Animals from both control groups received antigen-free doses. Two weeks after the boost, all animals (except the uninfected negative controls) were intranasally challenged with the SARS-CoV-2 Beta variant. Blood and tissue samples were collected after viral challenge on days 3 (n=6), 6 (n=6) and 14 (n=10 for S-21, S-29 and uninfected controls, and n=8 for S-2P) to study tissue damage and viral replication (Fig. 12a). All mice that developed severe disease (one mouse in the S-2P and S-21 groups, and all mice from the infected positive control group) were euthanized before day 14 following humane endpoints and were analyzed separately.

Anti-RBD (Fig. 12b) and anti-Spike (Fig. 16a) IgG humoral responses were evaluated prior to each immunization and viral challenge, and in those animals euthanized after infection on days 3, 6, and 14, or due to humane endpoints. Regardless of the immunogen used, all vaccinated animals developed similar anti-RBD (Fig. 12b) and anti-Spike IgG titers (Fig. 16a) that increased after each immunization and viral challenge (p<0.01, Connover-Iman test). Since the inventor did not identify significant differences in the humoral responses among the vaccinated groups after challenge (Fig. 16b and c), the inventor pooled these mice in a single "Post-challenge" group to simplify the analysis. Of note, unvaccinated but challenged positive controls elicited low levels of anti-Spike and anti-RBD IgG antibodies (Fig. 12b and Fig. 16a) that were only detected following day 6 after viral challenge (Fig. 16b and 1c).

Sera neutralizing activity against SARS-CoV-2 WH1, Beta, Delta, and Omicron VoCs was detected in all three vaccinated groups (Fig. 12c, d, e, and f). Interestingly, despite having similar levels of anti-RBD IgGs (Fig. 12b) and a slightly higher levels of anti-Spike IgG antibodies (Fig. 16a), S-2P vaccinated mice showed lower sera neutralizing activity against WH1 and Delta VoCs at day 3 than those immunized with S-21 (Fig 12c and e) (WH1 p<0.05; Delta p=0.052, Connover-Iman test). Sera neutralizing activity against Delta and Beta VoCs increased over time in the S-2P vaccinated group after viral challenge (Beta p<0.05; Delta p=0.052, Connover-Iman test), suggesting that infection may boost the humoral response in these animals. In line, unvaccinated mice developed low level of sera neutralizing activity against the SARS-CoV-2 Beta variant (Fig 12d) with limited cross-neutralizing activity against other VoCs (Fig. 12c, 12e, and 12f) after virus challenge. No boost effect on sera neutralizing activity was detected in S-21- and S-29-immunized mice after challenge, suggesting that the humoral response is reaching a plateau in these groups.

To determine whether S-2P-, S-21-, and S-29-vaccinated mice were protected against SARS-CoV-2-induced severe disease body weight changes (Fig. 12g) and survival after viral challenge (Fig. 12h) were analyzed. A progressive weight loss was observed in all unvaccinated but challenged mice starting on day 2 post-challenge. These mice developed a severe disease on days 5-9 and were euthanized following humane endpoints. Conversely, all vaccinated mice (except one S-2P- and one S-21-vaccinated mice), were disease-free (Fig. 12h) and did not experience weight loss. All mice belonging to S-29 group were protected from severe disease development (Fig. 12g and 12h).

The presence of SARS-CoV-2 in oropharyngeal swab and tissue samples from nasal turbinate, lung, and brain was analyzed by RT-qPCR. All vaccinated mice presented a significantly lower level of genomic viral RNA (gRNA) in lung at the beginning of infection (day 3) compare to the infected positive group (p<0.05, Peto & Peto Left-censored k sample test) (Fig. 13a). Most notably gRNA was scarcely detected in brain of vaccinated mice compared with unvaccinated animals (Fig. 13a). Interestingly, S-21- and S-29-vaccinated mice showed lower viremia in nasal turbinate than S-2P and control groups on day 3, and than S-2P vaccinated mice on day 6 (p<0.05, Peto & Peto Left-censored k sample test) (Fig. 13a). The lack of differences with the control group on day 6 could be explained due to the small number of unvaccinated mice that reached this timepoint, since the majority had been euthanized by this point (Fig 12h). Similarly, S-21 and S-29 groups exhibited lower viral loads in oropharyngeal swaps than unvaccinated mice (S-21 p<0.05; S-29 p=0.066; Peto & Peto Left-censored k sample test) (Fig. 13a). Generally, gRNA decreased overtime in all immunized mice regardless of the analyzed sample, whereas the opposite outcome was observed in mice belonging to the challenged control group, and in those vaccinated mice that developed severe disease (Fig. 13a). Similar results were observed when subgenomic viral RNA (sgRNA) was analyzed using the same biological samples (Fig. 16d).

To confirm active viral replication, Nucleoprotein (NP) levels were analyzed by immunohistochemistry (IHC). NP was hardly detected in lung and brain samples from S-2P, S-21 and S-29 groups (Fig. 13b). These data are according with the viral loads detected in these samples. Despite that, some tissue damage was still detected in the lung of all vaccinated groups. Remarkably, limited tissue damage was found in the brain of vaccinated mice, except in those animals euthanized due to humane endpoints (Fig. 13c).

Overall, S-2P, S-21, and S-29 trimers displayed an equivalent immunogenicity in K18-hACE2 mice and protected these mice from developing severe disease after SARS-CoV-2 Beta variant challenge. Interestingly, S-21- and S-29-immunized animals showed lower viremia in nasal turbinate than S-2P and infected controls on days 3 and 6 after challenge. The viral loads were also lower in oropharyngeal swabs of these mice on day 3 compared to infected control groups.

### Example 6: S-21 and S-29 trimer vaccination protects golden Syrian hamsters from COVID-19 development

To confirm the results obtained in K18-hACE2 mice, a second immunization and challenge experiment was performed using golden Syrian hamsters (GSH) with the same immunogens. Unlike K18-hACE2 mice, GSH develop a mild form of SARS-CoV-2-induced disease, from which spontaneously recover by day 14 after challenge (Sia, S. F. et al. Nature 583, 834-838 (2020); Brustolin, M. et al. Emerg Microbes Infect 12, 1-36 (2021)). GSH were immunized following a similar prime/boost strategy indicated for K18-hACE2 mice, intranasally challenged with the SARS-CoV-2 Beta variant and followed up until day 7 post-challenge (Fig. 14a). This time point was selected since it has been described that GSH start recovering themselves on such day (Sia, S. F. et al. Nature 583, 834-838 (2020); Brustolin, M. et al. Emerg Microbes Infect 12, 1-36 (2021)).

In accordance with K18-hACE2 data, the three vaccinated groups (S-2P, S-21 and S-29) developed similar levels of anti-RBD and anti-Spike binding IgG (Fig. 14b, and Fig. 17a and 17b). Interestingly, the second immunization did not boost vaccine-induced anti-Spike or anti-RBD IgG antibodies, indicating that a second vaccine dose might not be needed in this animal models. Interestingly, an increase in anti-Spike IgG levels was observed after viral challenge in vaccinated and in unvaccinated but challenged mice (Fig. 17a). However, when anti-RBD IgG responses were analyzed, that boosting effect was less evident and only detected in both the S-2P and in unvaccinated and challenged groups (Fig. 12b and Fig. 17b). These results suggest that viral challenge elicited a rapid humoral response in naive animals, boosting anti-Spike IgG responses but had a little effect in vaccinated GSH. Despite that, immunized GSH showed higher levels of anti-Spike and anti-RBD antibodies than challenged controls (p<0.001 for S-2P and Spike 29 group, and p<0.01 for Spike 21 group; Friedman test) (Fig. 14b and Fig. 17a).

Sera neutralizing activity was detected in vaccinated animals in all post-challenge time points against SARS-CoV-2 WH1, Beta and Delta, and to a lesser extent, Omicron variants (Fig. 14c, 14d, 14e and 14f). No differences were identified among immunized groups. Remarkably and contrarily to K18-hACE2 vaccine study data, sera neutralizing activity against all four SARS-CoV-2 variants were observed in challenged positive controls by day 4 after challenge (Fig. 14c, 14d, 14e and 14f). These results indicate that cross-reactive neutralizing antibodies were generated in those animals. Unexpectedly, the neutralizing activity against the SARS-CoV-2 Beta VoC was higher in challenged control animals than in S-21- and S-29-vaccinated GSH by day 7 (Fig. 14d). According to the binding ELISA data, neutralization titers also increased in immunized GSH by day 7 after viral challenge (p<0.05; Conover-Iman test), indicating that infection boosts vaccine-induced humoral neutralizing responses (Fig. 14c, 14d, 14e and 14f).

To determine whether vaccination protected GSHs from SARS-CoV-2-induced disease, animal weight overtime after viral challenge was monitored (Fig. 14g). Challenged control mice showed a progressive weight reduction until day 6 indicative of disease progression. One animal from this group showed clinical manifestation of SARS-CoV-2-induced severe disease and was euthanized according to humane endpoints (Fig. 14h). No weight loss was observed in vaccinated GSH, indicating that these animals were protected from disease development (Fig. 14g and 14h).

Viral replication in tissues was investigated determining the levels of genomic RNA (gRNA) and sgRNA by RT-qPCR. No differences among study groups in the levels of gRNA and sgRNA in nasal turbinate, lung, and in oropharyngeal samples were detected (Fig. 15a and Fig. 17c). However, vaccinated animals exhibited a decreasing trend in their nasal turbinate levels of both gRNA and sgRNA overtime after challenge (p=0.061; Peto & Peto Left-censored k sample test) (Fig. 15a and Fig. 17c). In addition, the analysis of nasal turbinate samples on day 7 post-challenge showed that vaccinated GSH displayed a lower gRNA and sgRNA levels tendency compared with unvaccinated-challenged controls (gRNA:p=0.061; sgRNA: p=0.056; Peto & Peto Left-censored k sample test) (Fig. 15a and Fig. 17c). To confirm RT-qPCR data, the presence of nucleoprotein (NP) was analyzed in nasal turbinate and lung by IHC. NP was not detected in nasal turbinate samples from immunized animals on day 7 (Fig. 15 b). These results confirm the decreasing trend observed overtime when gRNA and sgRNA were analyzed. Similarly, SARS-CoV-2 replication associated lesions were hardly detected in nasal turbinate samples on day 7 (Fig. 15c). However, despite NP was not detected in lung of vaccinated GSHs on day 7, low levels of tissue lesions were still present (Fig. 15c). No differences in tissue damage were observed in lung samples among study groups.

Overall, these results confirm that all three S-2P, S-21 and S-29 immunogens showed an equivalent immunogenicity and prophylactic activity in GSHs, protecting these animals from the development of severe SARS-CoV-2-induced disease.

## Claims

1. A polypeptide comprising a region from a SARS-CoV-2 spike protein containing at least one mutation selected from the group consisting of: V987H, L849K, S758E, K947R, Q755R, T961E, V963E, D985L, S982F, L517Y, G416R, K386R, V987W, D427I, D420R, A372W and D420Y, relative to the SARS-CoV-2 spike protein sequence as defined in SEQ ID NO: 77, or a functionally equivalent or immunogenically active variant thereof.

2. The polypeptide according to claim 1 comprising one of the following sets of mutations:
- A372W and D420R;
- D198F, G232L, A372W, N394Q and D420R
- Q755K, L849K, A892N, K947R and K1045R and
- S758E, T912R and K947R.

3. The polypeptide according to claim 1 comprising one of the following set of mutations:
- A706Q, S758E, L849K, V963E, S1030R and Q1113R;
- S758E, L849K and V963E;
- Q755K, C851Q and T961E;
- A706Q, S1030R and Q1113R; and
- S758E, T912R and K947R.

4. The polypeptide according to claim 1, wherein the polypeptide comprises:
- the V987H mutation and a K986P mutation relative to the SARS-CoV-2 spike protein sequence as defined in SEQ ID NO:77
- The L849K mutation and the K986P and V987P mutations relative to the SARS-CoV-2 spike protein sequence as defined in SEQ ID NO:77 and
- The S758E mutation and the K986P and V987P mutations relative to the SARS-CoV-2 spike protein sequence as defined in SEQ ID NO:77
or the polypeptide according to claim 3 which further comprises the K986P and V987P mutations relative to the SARS-CoV-2 spike protein sequence as defined in SEQ ID NO: 77.

5. The polypeptide according to any of claims 1 to 4 wherein the naturally occurring furin cleavage site in the SARS-CoV-2 spike protein has been mutated to avoid said site being recognized by a protease.

6. The polypeptide according to any of claims 1 to 6 comprising a sequence selected from the group consisting of SEQ ID NO:1 to 21

7. The polypeptide according to any of claims 1 to 7 further comprising a trimerization motif located C-terminally.

8. The polypeptide according to any of claims 1 to 7 further comprising at least an affinity tag.

9. The polypeptide according to any of claims 1 to 8 further comprising a target site for a protease.

10. The polypeptide according to any of claims 7 to 9 wherein
- if the polypeptide comprises a trimerization motif, the affinity tag is located C-terminally with respect to trimerization motif or
- if the polypeptide comprises a trimerization motif, an affinity tag and a target site for a protease, then the target site for a protease is located in between the trimerization motif and the affinity tag.

11. The polypeptide according to any of claims 1 to 10 wherein the polypeptide does not contain the transmembrane region (TM) and/or the C-terminal tail (CT) of the SARS-CoV-2 spike protein.

12. The polypeptide according to any of claims 1 to 11 comprising a sequence selected from the group consisting of SEQ ID NO:28 to 47 or 103 to 123..

13. A trimeric polypeptide which is formed by three monomer polypeptides, wherein each monomer polypeptide is as defined in any of claims 1 to 12 and wherein each of the monomer polypeptides comprises a trimerization motif.

14. A polypeptide according to any of claims 1 to 12 or a trimeric polypeptide according to claim 13 for use in the prevention or treatment of a disease caused by the infection by SARS-CoV-2.

15. A method for the detection of the presence of antibodies specific for the SARS-CoV-2 spike protein in a sample comprising the steps of:
(i) contacting the sample with polypeptide according to any of claims 1 to 12 or the trimeric polypeptide according to claim 13 and,
(ii) detecting the formation of a complex between antibodies specific for the SARS-CoV-2 spike protein present in the sample and the polypeptide or trimeric polypeptide of step (i).
